(19)

Europäisches Patentamt
European Patent Office
Office européen des brevets

(11) **EP 1 435 231 B1**

(12) **EUROPEAN PATENT SPECIFICATION**

(45) Date of publication and mention
of the grant of the patent:
**30.12.2009  Bulletin 2009/53**

(51) Int Cl.:
*A61K 9/127* (2006.01)    *A61K 31/704* (2006.01)

(21) Application number: **03258252.0**

(22) Date of filing: **31.12.2003**

(54) **Non-pegylated long-circulating liposomes**

Nicht-pegylierte lang-zirkulierende Liposome

Liposomes non-pegylés à longue durée de circulation

(84) Designated Contracting States:
**AT BE BG CH CY CZ DE DK EE ES FI FR GB GR
HU IE IT LI LU MC NL PT RO SE SI SK TR**

(30) Priority: **31.12.2002  IN MU11012002**

(43) Date of publication of application:
**07.07.2004  Bulletin 2004/28**

(73) Proprietor: **Bharat Serums & Vaccines Ltd.
Thane 400 604, Maharashtra (IN)**

(72) Inventors:
 • **Daftary, Gautam Vinod
  400 604 Thane
  Maharashtra (IN)**
 • **Pai, Srikanth Annappa
  400604 Thane (IN)**
 • **Rivankar, Sangeeta Hanurmesh
  400604 Thane (IN)**

(74) Representative: **Burford, Anthony Frederick
Beck Greener
Fulwood House
12 Fulwood Place
London
WC1V 6HR (GB)**

(56) References cited:
**WO-A-85/00968     WO-A-88/06442
WO-A-91/05546     US-A- 5 316 771**

 • **ZHIGALTSEV I V ET AL: "Liposomes containing
dopamine entrapped in response to
transmembrane ammonium sulfate gradient as
carrier system for dopamine delivery into the
brain of parkinsonian mice" JOURNAL OF
LIPOSOME RESEARCH, vol. 11, no. 1, February
2001 (2001-02), pages 55-71, XP001102782 ISSN:
0898-2104**
 • **FONSECA M J ET AL: "Doxorubicin induces
aggregation of small negatively charged
liposomes" EUROPEAN JOURNAL OF
PHARMACEUTICS AND BIOPHARMACEUTICS,
vol. 43, no. 1, 1997, pages 9-17, XP004256853
ISSN: 0939-6411**

**Description**

FIELD OF THE INVENTION

The present invention relates to liposomes, that can be used to contain and deliver diagnostic or therapeutic agents, and the manufacture thereof.

BACKGROUND OF THE INVENTION

Liposomes are commonly composed of phospholipid and/or sterols and consist of a vesicular structure based on lipid bilayers surrounding aqueous compartments. They vary widely in their physicochemical properties such as size, surface charge, and phospholipid composition.

Liposomes have received increasing attention as possible carriers for diagnostic or therapeutic agents. For example, liposomes have been used to deliver diagnostic agents such as contrast agents for magnetic imaging such as Gd:diethylenetriaminepentaacetic acid chelate (Gd-DTPA) (See e.g. U.S. Patent No. 6,132,763) and therapeutic agents such as anthracycline agents, which have been shown to exhibit marked activity against a wide variety of neoplasms. See e.g. U.S. Patent No. 4,769,250).

However, liposomes cause aggregation in the blood by their mutual reaction with various blood plasma proteins and are captured by the reticuloendothelial system (RES). For example, Kupffer's cells in the liver or fixed macrophages in the spleen take up the liposomes before they can reach their intended target. Capture by the RES has rendered selected delivery of the liposomes to target tissues or cells very difficult.

In addition to capture by the RES, the liposomes are subject to electrostatic, hydrophobic, and Van der Waals interactions with plasma proteins. These interactions result in destabilization of the liposomes leading to rapid clearance of the vesicles from circulation, often before reaching their target.

Also, in addition to cellular and protein interactions with the liposomes, difficulties have arisen in producing liposome encapsulating certain drugs because of the drugs' interactions with the phospholipids of the liposomes. For example, anthracyclines have exhibited a surfactant or detergent-like effect on the phospholipid vesicle bilayer that causes leakage and creates liposome vesicle instability. Thus, liposomes unstable to the circulation environment and/or its content will leak the antineoplastic agent prematurely to reaching the tumour site. As a result of the "leaky" liposomes and the resulting devastating toxicities, scientists have tried to develop long-circulating liposomes that are able to extravasate to tumour sites, which are highly vascular in nature.

Since most commonly used anti-cancer drugs are not specifically toxic to tumour cells and are toxic to all tissues they contact, they create undesirable side effects as a result of their interactions with normal tissues. For example, doxorubicin hydrochloride is one of the most commonly used cytotoxic anthracycline antibiotics used in cancer chemotherapy and has been shown to have activity against a wide variety of neoplasms. Doxorubicin hydrochloride is effective in the treatment of many solid tumours and leukaemias. It is particularly effective in the treatment of breast cancers involving polytherapies. Doxorubicin hydrochloride is protocol therapy for AIDS related Kaposi's sarcoma. Doxorubicin hydrochloride also has notable activity against tumours of the ovaries, lung, testes, prostate, cervix, head and neck, oestrogenic sarcomas and Ewing's sarcoma.

Conventional compositions of doxorubicin hydrochloride injection are available as freeze-dried product or as a solution of doxorubicin hydrochloride in water. Freeze dried product is required to be reconstituted with Water for Injection before administration. Both of these marketed products have been associated with a number of toxicities when administered intravenously. Severe myelosuppression is usually dose limiting. Other toxicities include nausea and vomiting, alopecia, mucositis (including stomatitis and esophagitis) and cardiotoxicity, which may limit doxorubicin hydrochloride use. Doxorubicin hydrochloride is a potent vesicant that may cause extravasation and necrosis at the injection site or at any site that the skin is exposed. "Doxorubicin flare" is not uncommon and is characterized by erythematous streaking at the injection site. "Doxorubicin flare" usually subsides in about a half an hour.

The mechanism of action of doxorubicin hydrochloride is not known exactly but many possibilities have been studied and described. The primary mechanism involves the ability of doxorubicin hydrochloride to intercalate DNA. The integrity of the DNA is significantly compromised and commonly results in altered DNA functions. Single and double strand brakes are also common due to doxorubicin hydrochloride intercalation with DNA. Another mechanism of doxorubicin hydrochloride involves its ability to generate free radicals that induce DNA and cell membrane damage. Doxorubicin hydrochloride also inhibits topoisomerase II, rendering the reproduction of DNA ineffective.

Some of the resulting toxic affects of doxorubicin hydrochloride include cardiac toxicity, anaphylactic reaction, emetogenicity, myelosuppression, muccocytis, skin toxicity, alopecia, and toxicity to the injection sight (Cancer Investigation, 19 (4): 424-436 (2001)). In theory, prolonged circulation systems (slow release) that effectively deliver and release a drug to tumours and the near vicinity of tumour cells are more advantageous. Thus, it is desirable to have a stable liposome capable of encapsulating agents, such as doxorubicin hydrochloride, that do not prematurely release their contents to healthy or non-cancerous tissues.

Several approaches taken in an effort to increase the circulation time of liposomes and thus ensure delivery of the liposome contents to the target tissue include the following: masking the liposomes from the reticuloendothelial system recognition using a sialic acid residue coating (U.S. Patent No. 4,501,728); rigidifying the liposome membrane with sphingomyelin or neutral phospholipid with predominantly saturated acyl chains containing 5 to 20% glycolipid (U.S. Patent No. 4,920,016); forming liposomes with a 3-80 fold higher drug to lipid ratio than traditional liposome preparations in a 3-compartment system of the agent, bilayers, and release inhibiting buffer containing citric acid (U.S. Patent No. 6,083,530); incorporating cholesterol in the liposome (Alberto A. Gabizon, Cancer Investigation, 19(4) 424-436 (2001)); and derivatizing the phospholipid with polyethylene glycol (pegylated liposomes) (U.S. Patent Nos. 5013556 and 6132763).

Unfortunately, the above approaches have shown only limited potential to extend the circulation time of the liposomes *in vivo.* For example, it has been determined that masking the liposome with sialic acid only had limited ability to extend the circulation half lives of *in vivo* liposomes (U.S. Patent No. 4,920,016). To overcome these problems, scientists have coated the liposome surface with a hydrophilic polymer such as polyethylene glycol (PEG) to prevent adsorption of various blood plasma proteins to the liposome surface. See e.g. U.S. Patent No. 5,013,556, and U.S. Patent No. 5,676,971. These pegylated liposomes have been called sterically stabilized liposomes or stealth liposomes. The pegylated liposomes appeared to reduce some of the toxic effects caused by the release of their contents, but, unfortunately, new toxic effects appeared because of the presence of the polyethylene glycol. For example, the liposomal preparations containing pegylated phospholipids have lead to skin toxicity generally known as "Hand-Foot syndrome," which results in skin eruptions/ulcers on the palms of the hands and soles of the feet (Kenneth B. Gordon, Cancer, Vol. 75(8), 1995, 2169-2173).

Another disadvantage with pegylated liposomes is the presence of large molecules (PEG) on the liposomal surface may reduce the interactions of liposomes with cells and hinder entry of liposomes into the tumour tissue, thereby possibly reducing the accumulation of liposomal drug in the tumour tissue. (Clinical Cancer Research, (5), 1999, 3645-3652).

Thus, there remains a need for stable, long circulating liposomes that do not cause such deleterious effects such as the "Hand-Foot syndrome" as well as methods of manufacturing such liposomes and compositions based on them. The present invention meets this need.

Zhigaltsev et al (J. Liposome Res. 11 (1):55-71) reports a study of the active loading of liposome with dopamine in response to an ammonium sulphate gradient. The initial stage of the liposome preparation involved the hydration of a dry lipid film formed by evaporation of a chloroform solution of phosphatidylcholine (ePC, hsPC or DMPC) and cholesterol. The hydration was conducted with 120 mM ammonium sulphate solution to provide a final lipid concentration of 20, 50 or 100 mg/ml. There is a reference to establishing a transmembrane ammonium sulphate gradient by dialysis exchange against sucrose but no disclosure of the use of sucrose in the ammonium sulphate hydration solution.

WO-A-8806442 relates to the encapsulation of doxorubicin or other antineoplastic agents in a liposome using a transmembrane ion gradient to load the agent. Reference is made to the hydration of phospholipid/cholesterol lipid films but not to the use of a hydrating solution comprising ammonium sulphate or sucrose.

WO-A-8500968 relates to the reduction in toxicity of adriamycin (4' O-tetrahydropyranyl doxorubicin) or other drugs by encapsulation in a liposome containing α-tocopherol or other drug-protective compound. Reference is made to the evaporation of a chloroform/methanol solution of a phospholipids, cholesterol, adriamycin and α-tocopherol and hydration of the resultant film with a phosphate buffered saline solution. The amount of hydrating solution used is 1 ml/ 60 μmoles lipid. There is no reference to the use of a hydrating solution comprising ammonium sulphate or sucrose.

US-A-5316771 discloses transmembrane loading of amphiphatic drugs into liposomes using an ammonium transmembrane gradient. In the exemplified processes, a lipid film is hydrated with aqueous ammonium sulphate containing desferal (desferoxamine mesylate). In each case 5 ml of the solution was added to a film formed from 100 mg egg phosphatidylcholine. There is no disclosure of the use of sucrose in the ammonium sulphate hydration solution.

US-A-4235871 relates to the preparation of loaded liposomes by emulsifying a mixture of an organic solution of a lipid with an aqueous solution of the active, removing the organic solvent to provide a gel and then suspending the gel in water. In exemplified processes, a lipid formed from 50 μmole phospholipid and 50 μmole sterol is dissolved in an organic solvent and mixed with 10.5 ml of an aqueous solution of the active and, after emulsification, 5 ml of a further aqueous solution is added to the resultant gel. Reference is made to the presence of sucrose but not as a component of an aqueous hydration media and there is no reference to ammonium sulphate.

EP-A-0561424 relates to the use of sucrose as a protective sugar during liposome dehydration. There is a reference to the addition of aqueous buffer to solution of a lipid formed from egg phosphatidylcholine but no reference to ammonium sulphate.

The principal object of the present invention is to develop a liposomal doxorubicin composition that will have a long circulation time and that will not give rise to Hand-Foot-Syndrome. Another object of the present invention is to decrease the toxicity and other adverse effects associated with the administration of doxorubicin such as nausea, vomiting, and alopecia. Yet another object of the present invention is to develop liposomes to support such compositions of antineoplastic agents such as doxorubicin.

SUMMARY OF THE INVENTION

According to one aspect, the present invention provides a process for the manufacture of long circulating non-pegylated liposomes comprising dissolving one or more phospholipids and one or more sterols in a solvent or mixture of solvents and removing said solvent(s), wherein, before or after solvent removal, the resultant lipids are hydrated with an aqueous hydration medium in an amount in the range of 10 to 35 ml for each mmole of phospholipid present in the lipid solution, wherein the aqueous hydration medium comprises sucrose and not less than 125mmoles/litre ammonium sulphate.

Preferably the amount of aqueous hydration medium used is about 30ml for each mmole of phospholipid in the lipid solution.

The process usually further comprises sizing the liposomes in the liposomal composition to 0.06μm to 0.16μm and/or removing the extra-liposomal hydration salt from the liposomal composition using dialysis buffer solution to form sized non-pegylated liposomes

The process of manufacture of the non-pegylated liposomes may further comprise loading the liposomes with a therapeutic or diagnostic agent. Preferably the therapeutic agent is an antineoplastic agent such as daunorubicin hydrochloride, epirubicin hydrochloride and, especially, doxorubicin hydrochloride.

Preferably the molar ratio of phospholipid to sterol is from 1:0.1 - 1:2 and is more preferably about 1:0.7.

Preferred phospholipids have a phase transition temperature of 40˚C to 60˚C, have a fatty acid chain with a minimum of sixteen carbons and are selected from distearoyl phosphatidylcholine (DSPC), dipalmitoyl phosphatidylcholine (DPPC), hydrogenated soya phosphatidylcholine (HSPC) and derivatives of such phospholipids. Preferably, the phospholipid is distearoyl phosphatidylcholine (DSPC) and the sterol is cholesterol.

The process may also involve sizing of the non-pegylated liposomes. They are preferably sized by extrusion through a filter having a pore size of 0.4 to 0.05 μm.

Another embodiment of the present invention provides for liposomes manufactured by the process described and claimed herein. The liposomes comprise the ingredients in the proportions described in the process for the manufacture thereof and preferably the average size of liposomes so obtained is 0.06μm to 0.16μm.

The present invention also provides a long circulating non-pegylated liposomal doxorubicin composition for parenteral administration comprising non-pegylated doxorubicin liposomes, histidine hydrochloride, and sucrose; wherein the non-pegylated doxorubicin liposomes are obtainable by the process of the invention and comprise a phospolipid; especially distearoylphosphatidyl choline; a sterol, especially cholesterol; and sucrose; wherein the liposomes preferably have an average particle size of 0.06 to 0.16 μm. Such non-pegylated doxorubicin liposomes typically have a circulation time in blood at least 25 times longer than that obtained with Adriamycin® when tested in swiss albino mice at equivalent doses.

Preferably doxorubicin concentration (calculated as hydrochloride) is from 1 to 10 mM, more preferably from 3nM to 7nM and most preferably about 3.45mM.

The molar ratio of distearoylphosphatidyl choline to cholesterol is preferably from 1:0.6 to 1:0.8; more preferably about 1:0.7.

The molar ratio of doxorubicin (as hydrochloride) to distearoylphosphatidyl choline is preferably from 1:2 to 1:15, more preferably 1:2 to 8 and most preferably about 1:3.5.

The sucrose concentration is preferably from 0.1M to 0.5M, more preferably 0.25M to 0.3M, especially about 0.27M.

The concentration of histidine hydrochloride is preferably from 1mM to 100mM, more preferably from 8 to 12 mM, and most preferably about 10mM.

The preferred average size of the liposomes is from 0.08μm to 0.12μm.

In an exemplary composition, the doxorubicin (as hydrochloride) is present at about 2mg/ml; and the molar ratio of doxorubicin to phospholipid is about 1:3.5; and the ratio of phospholipid to cholesterol is about 1:0.7.

In another exemplary composition, the doxorubicin (as hydrochloride) is present at about 4mg/ml and the molar ratio of doxorubicin to phospholipid is about 1:3.5 and the ratio of phospholipid to cholesterol is about 1:0.7. The circulation time (t½) of the composition in blood is preferably more than 40 times longer than that obtained with Adriamycin when tested in swiss albino mice at equivalent doses.

DETAILED DESCRIPTION OF THE INVENTION

The present invention provides stable, long circulating non-pegylated liposomes, as well as a method of manufacture thereof. Pegylated liposomes are liposomes coated with polyethyleneglycol (PEG). The surface of the liposome is decorated with several thousand strands of PEG, a process called "pegylation". The PEG strands make the surface of the liposome "hairy" and this prevents the rapid absorption of the liposomes to the surface of blood proteins. The rapid absorption accelerates the rapid removal from blood of liposomes. In contrast, the pegylated liposomes are protected and are removed from blood at a much slower rate. Compared with liposomes made without PEG, pegylated liposomes are more stable and are less extensively taken up by cells of the reticuloendothelial system (RES), and have a reduced tendency to leak any encapsulated agent or drug while in circulation. For example, the pharmacokinetics of PEG-liposomes encapsulating doxorubicin is characterized by a long circulating half-life, slow plasma clearance, and a reduced volume of distribution compared with non-pegylated liposomal doxorubicin or free doxorubicin. The long circulation and ability of pegylated liposomes to extravasate through tumour vasculature results in localization of doxorubicin in tumour tissue with the increased possibility of increased tumour response because of enhanced drug accumulation especially in highly angiogenic tumours. Also, the increased stability of pegylated liposomes over conventional liposomes results in a decrease in availability of drug in the tissue of sensitive organs and thereby a decrease in toxicity and other adverse effects such as nausea, vomiting, and alopecia. However, a serious side effect known as "Hand-Foot syndrome", where skin eruptions or ulcers have been observed on the palms of the hands and soles of the feet, has been reported to result from clinical uses of the pegylated liposomes (Kenneth B. Gordon, Cancer, Vol. 75(8), 1995, 2169-2173). Another disadvantage with pegylated liposomes is that the presence of large molecules (PEG) on the liposomal surface may reduce the interactions of liposomes with cells and hinder entry of liposomes into the tumour tissue, thereby possibly reducing the accumulation of liposomal drug in the tumour tissue.

The process of the present invention provides stable, long circulating, low toxicity non-pegylated liposomes that exhibit the stability of the pegylated liposomes with the long circulation half-life and reduced toxicity described above. However, since the liposomes of the present invention do not require the use of PEG to achieve the above results, they do not cause "Hand-Foot syndrome".

In the process of the present invention, hydration of the lipids is carried out, using an aqueous hydration medium comprises ammonium sulphate and sucrose before or after evaporation of the solvent used to dissolve the lipids. Solvents suitable to the invention are organic solvents in which the phospholipid can be dissolved. One skilled in the art would appreciate commonly used and suitable solvents in the manufacture of liposomes. Exemplary suitable solvents include, but are not limited to, chloroform, methylene chloride, ethanol, methanol or acetone.

When the hydration of lipids is carried out after evaporation of the solvent, solvents such as chloroform or methylene chloride are preferred.

When the hydration of lipids is carried out before evaporation of the solvent, water miscible solvents such as ethanol, methanol, or acetone are preferred.

When hydration is carried out after evaporation of the solvent, the process comprises forming a lipid film by evaporating the solvent from a lipid solution comprising one or more phospholipids, one or more sterols, and a solvent or a mixture of solvents.

Evaporation of a solvent can be accomplished by any evaporative technique such as, but not limited to, evaporation by passing a stream of inert gas over the solution, by heating, by vacuum, or by heating under vacuum. Commonly, rotary evaporator flasks are employed.

When the hydration is carried out before evaporation of the solvent, the process comprises evaporation of the solvent from the solvent-containing aqueous liposomal suspension. Evaporation of a solvent can be accomplished by any evaporative technique such as, but not limited to, evaporation by passing a stream of inert gas over the solution, by heating, by vacuum, or by heating under vacuum. Commonly, rotary evaporator flasks are employed.

After a solvent or mixture of solvents is evaporated, only the liposomes remain in the aqueous suspension form.

Any phospholipid suitable to prepare liposomes may be used in the present invention. Suitable phospholipids include those that tend to decrease permeability of the liposomal membrane. Liposomes containing phospholipids with long fatty acid chains are more stable and result in a slower release of agent than liposomes comprised of phospholipids having shorter fatty acid chains. As the carbon chain length of the fatty acid increases, the phase transition temperature also increases. Liposomes comprised of phospholipids with higher phase transition temperature release their contents slower than liposomes comprised of lower phase transition temperature phospholipids. Higher phase transition temperatures enable slow release of the contents from inside the liposomes into the blood stream as the phospholipid membranes are semipermeable. Other phospholipid characteristics that effect membrane permeability and stability include degree of saturation and charge.

Preferably, liposomes of the present invention contain neutral lipids. It is preferred that the neutral lipids have a phase transition temperature of 40°C to 65°C and more preferably of 50°C to 54°C. Preferable phospholipids have a fatty acid chain of at least sixteen carbons.

Suitable phospholipids employed in the process of the present invention, include but are not limited to distearoyl phosphatidylcholine (DSPC), dipalmitoyl phosphatidylcholine (DPPC), hydrogenated soya phosphatidylcholine (HSPC) or derivatives of such phospholipids. Phosphatidylcholines are preferred neutral lipids. A preferred phospholipid is 1,2-distearoyl-sn-glycerol-3-phosphocholine, which is commonly known as distearoyl phosphatidylcholine (DSPC). The molecular weight of DSPC is 790 and it has the molecular formula of $C_{44}H_{88}NO_8P$.

Sterols are incorporated into liposomes along with phospholipids to alter rigidity and permeability of liposome membranes. An exemplary sterol is cholesterol and derivatives or analogues thereof. Cholesterol tends to increase rigidity and decrease permeability of liposomal membranes. Cholesterol is an amphipathic molecule and inserts itself into the phospholipid membrane with its hydroxyl groups orientated towards the aqueous surface. Cholesterol is incorporated in a concentration that provides optimum permeability to the liposome membrane, but also maintains the rigidity of the membrane. The selection of phospholipid to cholesterol ratio defines the rate of dissolution of the contents from the liposomes. Liposomes of the present invention usually have a molar ratio of phospholipids to sterol ranging from 1:0.1 to 1:2. Preferably the range is 1:05 to 1:1.5. A preferable molar ratio of phospholipids to sterol when distearoyl phosphotidyl choline (DSPC) is the phospholipid and cholesterol is the sterol is from 1:0.6 to 1:0.8. A preferred molar ratio is about 1:0.7.

The solvent or mixtures of solvents are evaporated under vacuum. When the hydration is carried out after solvent removal, the lipid film formed is hydrated with an aqueous hydration medium to form liposomes. The aqueous hydration medium is added to the film with agitation or under mixing to hydrate the lipid film and form liposomes. One skilled in the art would appreciate suitable aqueous hydration media to employ. Preferable aqueous hydration media contain buffers/salts so as to be available to establish a chemical gradient later in the process to assist in loading various agents into the liposomes.

The volume of the aqueous hydration medium is controlled/reduced as compared to amounts of hydration medium used in conventional liposome and pegylated liposome manufacture. By reducing the volume of aqueous hydration medium, the phospholipids can pack tighter together resulting in a thicker liposome membrane or "shell". The thicker "shell" provides for stable, long-circulating, slow release and decreased toxicity of the liposome contents without the need for PEG. The smaller the volume of hydration medium used, the tighter the phospholipids will pack together and the thicker the shell will become. By "controlled/reduced" it is meant that the volume of aqueous hydration medium used in the present invention is less than previously known or accepted amounts of aqueous hydration medium. Using a preferred reduced volume of hydration medium (e.g. 30ml for each mmole of phospholipid) and a preferred concentration of cholesterol, the resulting liposomal composition would have a rigid phospholipid bilayer.

This reduction in hydration medium volume can also be viewed in terms of the ratio of volume of medium used per moles of phospholipid present in the lipid solution. In the present invention, the amount of aqueous hydration medium used is in the range of 10 to 35 ml for each mmole of phospholipid present in the lipid solution. Preferably the volume of aqueous hydration medium is between 20-35 ml, especially 20 to 30 ml, for each mmole of phospholipid present in the lipid solution. More preferably, the volume of aqueous hydration medium is about 30ml for each mmole of phospholipid used in the lipid solution.

Liposomes are sized appropriately. One skilled in the art would appreciate known methods of liposome sizing. Homogenization under pressure is one such method. Another suitable method includes extruding the liposomes through filters with a pore size to match the desired liposome size. Because the liposomes of the present invention have a tighter packed membrane, sizing tends to be more difficult than with conventional liposomes. Thus, they are preferably sized through a series of filters with increasingly smaller pore size. For example, following hydration, liposomes are initially passed through a filter having a pore size of $0.40\mu m$ followed by successively smaller pore sized filters of $0.06\mu m$ or, preferably, 0.05. The resulting liposomes range in size from 0.05 or $0.06\mu m$ - $0.2\mu m$. A preferred average size range is $0.08\mu m$ to $0.12\mu m$.

Extraliposomal salt in the hydration medium is removed or washed from the liposomes. Dialysis using a dialysis medium is an exemplary method of removing extraliposomal hydration medium salt. Any suitable buffer solution may be used in the dialysis. Removal of extraliposomal salt present in the liposomal composition creates an inside-to-outside chemical gradient across the liposomal membrane, which is later called upon for loading of the liposomes. Other suitable means to remove the extraliposomal salt includes ultrafiltration or column chromatography.

The liposomes of the present invention provide a long circulating, slow release delivery mechanism for therapeutic or diagnostic agents. Any known method can be used to load the liposomes with a desired therapeutic or diagnostic agent. Exemplary methods include adding the agent to the lipid film before hydration of the lipid film, incorporating the agent directly into the hydration medium, by pH gradient, or by chemical gradient. A preferred method involves loading an agent using a chemical gradient. When the liposomes are loaded by active loading process, the drug solution is admixed with the blank liposomal suspension at a temperature higher than or equivalent to phase transition temperature of the phospholipids.

Using a chemical gradient, the amount of agent can be readily controlled and once the agent is loaded inside the liposome, the leakage into the extraliposomal medium is minimal. In addition, if a hydration medium containing a buffer/salts is used in the hydration step, the creation of such a gradient becomes very feasible after removing the extraliposomal hydration medium salt as described above. However, hydration with ammonium sulphate solution rendered isotonic with sodium chloride (as disclosed in US patent 5,316,771) results in liposomes which leak on storage. The free drug content of the liposomal composition increases on storage which in turn increases the toxicity. Hence there is a need to strengthen the liposomal membrane. The present invention thus provides the concomitant use of an iso-osmotic agent that is non-reactive with other ingredients of the solution and the liposomes themselves in the hydration medium. It was found that use of sucrose is protective for liposomal membranes. Sucrose helps in protecting and rigidifying the liposomal membrane and also to maintain the isotonicity of the liposomal composition. Liposomal membranes have been protected for dehydration before freeze-drying by use of saccharides such as trehalose, sucrose, or maltose (U.S. Patent 4,880,635).

The present invention thus provides using sucrose with ammonium sulphate as a hydration medium giving liposomes that are more rigid and that do not leak the agent encapsulated in them on storage. With the addition of sucrose to the hydration medium, sucrose remains inside and outside surface of the liposomal membrane hardening both sides of the liposomal membrane, thereby reducing the leakage of the drug. It is preferable that the concentration of sucrose in the hydration medium is 0.1M to 0.5M. A concentration of 0.25M to 0.3M, especially about 0.27M, is preferred.

The concentration of ammonium sulphate in the hydration medium plays a vital role on drug leakage from the liposomes. Ammonium sulphate in a concentration less than 125mM whenever used for hydration for forming liposomes showed the drug leakage on storage. Thus the concentration of ammonium sulphate solution is not less than 125mmole/ litre, and the hydration medium contains sucrose.

When dialysis is performed, it removes the extraliposomal salt, i.e. ammonium sulphate, but does not remove intra-liposomal ammonium sulphate, thus causing the inside-to-outside chemical gradient across the liposome membrane.

There are many suitable buffer solutions that can be used both to load the agent into the liposomes and to dilute the resulting liposomal composition to a desired concentration of the agent. Since liposomes primarily contain phospholipids, which are stable at around neutral pH of 6.0 to 8.0, buffer solutions used to load and dilute liposomes should also have a neutral pH. Also, ideally the buffer solution should be suitable for parenteral preparations. Some of the most common buffer solutions used in parenteral preparations, which are suitable in the present invention for loading the agent into the liposomes and for dilution of the liposomal composition, are glycine, phosphate, citrate, acetate, and histidine buffers. Histidine buffer solution is preferable it has the most stable pH in the neutral range. Preferably, the buffer solution comprises sucrose and histidine hydrochloride in a molar ratio of from 29:0.1 to 29:10, more preferably about 29:1. The histidine hydrochloride to sucrose weight ratio can be about 1:50. Use of sucrose helps in protecting and rigidifying the liposomal membrane and also in maintaining the isotonicity of the liposomal composition.

After the liposomes are loaded, any untrapped agent is removed. Suitable methods include, but are not limited to, gel filtration chromatography, dialysis, or treatment with microporous styrene/divinylbenzene copolymer resin, especially Dowex®, and subsequent filtration. Dowex® treatment is a preferred method because of its ease of use. When dialysis is used, it is preferably performed in the same manner as described above when removing extraliposomal hydration medium salts.

As discussed above, by controlling or reducing the amount of aqueous hydration medium, the resulting liposomes have an increased phospholipid content per unit volume as compared to conventional or pegylated-liposomes. Increase in phospholipid content increases liposome stability and decreases permeability, and thus slows the release of any entrapped agent.

Suitable agents for loading into liposomes of the present invention are watersoluble amphipathic compounds with ionizable groups. Amphipathic agents exhibit both hydrophilic and lipophilic characteristics and may be a therapeutic or diagnostic agent. Therapeutic agents may be any desired agent and include antineoplastic agents.

An antineoplastic agent is a drug that prevents, kills, or blocks the growth and spread of cancer cells. There are many suitable antineoplastic agents some of which include altretamine; asparaginase; BCG; bleomycin sulphate; busulfan; carboplatin; carmustine; chlorambucil; cisplatin-cis-platinum, cis-diammine-dichloroplatinum; cladribine, 2-chlorodeoxyadenosine; cyclophosphamide; cytarabine-cytosine arabinoside; dacarbazine imidazole carboxamide; dactinomycin; daunorubicin-daunomycin, daunorubicin hydrochloride; dexamethasone; doxorubicin, doxorubicin hydrochloride; epirubicin; etoposide-epipodophyllotoxin; floxuridine; fluorouracil; fluoxymesterone; flutamide; fludarabine; goserelin; hydroxyurea; idarubicin HCl; ifosfamide-isophosphamide; interferon alpha; interferon alpha 2a; interferon alpha 2b; interferon alpha n3; irinotecan; leucovorin calcium; leuprolide; levamisole; lomustine; megestrol; melphalan-L-phenylalanine mustard, L-sarcolysin; melphalan hydrochloride; mechlorethamine, nitrogen mustard; methylprednisolone, methotrexate-amethopterin, mitomycin-mitomycin-C; mitoxantrone; mercaptopurine, paclitaxel; plicamycin-mithramycin; prednisone; procarbazine; streptozocin-streptozotocin; tamoxifen; 6-thioguanine; thiotepa-triethylene thiophosphoramide; vinblastine; vincristine; or vinorelbine tartrate. Preferred antineoplastic agents include doxorubicin hydrochloride, daunorubicin hydrochloride, and epirubicin hydrochloride.

The present invention also provides for loading the liposomes with diagnostic agents including, but not limited to, MRI (magnetic resonance imaging) contrast agents (also called paramagnetic agents) used to help provide a clear picture during MRI. MRI is a special kind of diagnostic procedure that uses magnets and computers to create images or "pictures" of certain areas inside the body. Unlike x-rays, it does not involve ionizing radiation. Exemplary MRI diagnostic agents include gadodiamide; gadopentetate; gadoteridol; gadoversetamide, and Gd:diethylenetriaminepentacetic acid chelate (Gd-DTPA) (U.S. Patent No. 6,132,763).

Once liposomes are loaded and the unencapsulated therapeutic/diagnostic agent removed, the liposomal composition may be aseptically filtered for sterilization making it suitable for parenteral administration. Ideally the filter is at least a 0.2μm filter. The liposomal composition is then filtered into a sterile depyrogenated bulk container. Subsequently the sterile composition is filled aseptically into sterile depyrogenated smaller containers such as glass vials. The air in the headspace of the container is removed by purging with an inert gas, such as nitrogen and the containers are sealed. By "suitable for parenteral administration" it is meant that the composition is sterile, isotonic and controlled for bacterial endotoxins.

The present invention also provides for stable, long-circulating, low toxicity non-pegylated liposomes. The liposomes are preferably manufactured by the methods described herein. The liposomes of this invention are long circulating non-pegylated liposomes that have a blood circulation half-life of at least 25 times longer than conventional non-liposomal formulations (Adriamycin®) when tested in swiss albino mice at equivalent doses. A preferred blood circulation half-life is about 40 times longer than that obtained with Adriamycin®.

Non-pegylated liposomes of the present invention are comprised of a phospholipid and cholesterol. Acceptable ratios of phospholipid to cholesterol are described above and are preferably at a molar ratio of 1:0.1 to 1:2. A preferred molar ratio of phospholipid to sterol is about 1:0.7. Phosphatidyl cholines are preferred phospholipids and distearoyl phosphatidylcholine (DSPC) is especially preferred.

The non-pegylated liposomes may be loaded with a diagnostic or therapeutic agent. Such agents are known and discussed above. Non-pegylated liposomes of the present invention are preferably loaded using a chemical gradient as discussed above.

A preferred non-pegylated liposome of the present invention is loaded with doxorubicin hydrochloride and is prepared using methods described above. In one embodiment, when loading doxorubicin hydrochloride using the active loading procedure described above, the agent is dissolved in a suitable buffer solution (as described above) before loading to get a concentration of at least 25mM. When the active loading process involves an ammonium sulphate gradient, the ammonium sulphate reacts with doxorubicin hydrochloride to form doxorubicin sulphate. Doxorubicin sulphate is insoluble and remains inside the liposomes after loading. Once any unentrapped or free drug is removed from loaded liposomes, the drug-loaded liposomes are diluted using aqueous buffer solution to achieve the required drug concentration. The preferred buffer solution used is sucrose-histidine buffer solution as discussed previously.

An exemplary non-pegylated liposomal doxorubicin composition contains about 2mg/ml doxorubicin (calculated as hydrochloride). Another exemplary non-pegylated liposomal doxorubicin composition contains about 4mg/ml doxorubicin (calculated as hydrochloride). Using methods of the present invention, the doxorubicin may be loaded into non-pegylated liposomes at a concentration twice of that desired in the final desired composition. Then the loaded liposomes may be diluted with a suitable buffer solution (as described above) to achieve the desired concentration of doxorubicin per ml of liposomal composition. On dilution the external medium in which the liposomes are suspended is diluted, whereas the agent inside the liposomes remains undiluted.

In a preferred embodiment, the molar ratio of doxorubicin hydrochloride to phospholipids is from 1:2 to 1:15. A preferred molar ratio is about 1:3.5.

The present invention also provides non-pegylated liposomal doxorubicin compositions. The composition comprises non-pegylated liposomes as described above in suitable pharmaceutically acceptable carriers, which are known in the art. The liposomes have been loaded with doxorubicin hydrochloride. The compositions are suitable for parenteral administration, and are long circulating.

One embodiment provides long circulating non-pegylated liposomal doxorubicin compositions for parenteral administration. The liposomal composition comprises non-pegylated doxorubicin liposomes in a pharmaceutically acceptable carrier. Suitable pharmaceutically acceptable carriers are known in the art. In a preferred pharmaceutical composition, the concentration of doxorubicin varies from 1mM to 10mM, more preferably about 6.9mM but most preferably about 3.45mM. The molar concentration of phospholipids varies from 10mM to 15mM of the parenteral composition. A more preferred content is about 12.15mM.

The preferred composition further comprises distearoylphosphatidyl choline, cholesterol, histidine hydrochloride, and sucrose. Preferably the liposomes have an average size 0.06μm to 0.16μm.

Preferably the doxorubicin hydrochloride content is 1 - 10mM and more preferably the doxorubicin hydrochloride content is about 3.45mM.

In preferred compositions of the present invention, the molar ratio of distearoylphosphatidyl choline to cholesterol is from 1:0.6 to 1:0.8, and is preferably about 1:0.7.

In preferred compositions of the present invention, the molar ratio of doxorubicin hydrochloride to distearoylphosphatidyl choline is from 1:2 to 1:10, preferably from 1:2 to 1:8 and more preferably about 1:3.5.

The sucrose content is preferably from 0.45M to 0.55M, more preferably from 0.1M to 0.5M, especially 0.25M to 0.3M, and most preferably about 0.27M.

In preferred compositions of the invention the content of histidine hydrochloride is from 1mM to 100mM, more preferably from 8 -to 12 mM, and most preferably about 10mM.

In preferred compositions of the invention, the liposomes have an average size $0.08 \mu m$ - $0.12 \mu m$.

In one embodiment of the invention, the doxorubicin hydrochloride is present at about 4mg/ml, and the molar ratio of doxorubicin to DSPC is about 1:3.5, and the ratio of DSPC to cholesterol is about 1:0.7.

In another embodiment of the invention, the doxorubicin hydrochloride is present at about 2mg/ml, and the molar ratio of doxorubicin to DSPC is about 1:3.5, and the ratio of DSPC to cholesterol is about 1:0.7.

The doxorubicin liposomes in the compositions preferably have a half circulation time ($t_{1/2}$) more than 40 times longer than Adriamycin® when tested in swiss albino mice at equivalent doses.

Tumour growth is reduced by administering a therapeutically effective amount of a non-pegylated liposomal doxorubicin composition of the present invention. As the non-pegylated liposomal doxorubicin compositions have a prolonged circulation time, exhibit decreased toxicity and do not present "Hand-Foot Syndrome" issues, they provide a viable treatment for reducing tumour growth. A skilled practitioner would be able to use the data presented herein as well as common knowledge of dosage amounts, dosage times, and routes of administration, to treat an individual having a tumour susceptible to treatment by doxorubicin hydrochloride with the non-pegylated doxorubicin liposomes of the present invention. The 2mg/ml and 4mg/ml doxorubicin hydrochloride strength compositions of the present invention are useful for treatment of reducing tumour growth.

The present invention also provides a preferred process for making the preferred compositions of the invention. The process comprises

dissolving lipids comprising distearoylphosphatidylcholine (DSPC) and cholesterol in a single solvent or in a mixture of solvents,

removing said solvent(s) before or after hydrating the lipids by addition of an aqueous hydration medium to form liposomes in a liposomal composition, wherein said aqueous hydration medium comprises ammonium sulphate and sucrose and having an ammonium sulphate concentration of not less than 125mM, and wherein the aqueous hydration medium is added in an amount in the range of 10ml to 35ml per each mmole of DSPC;

sizing the liposomes in the resultant liposomal composition, preferably to $006 \mu m$ - $0.16 \mu m$;

removing extraliposomal ammonium sulphate from the sized liposomal composition using a sucrose - histidine buffer solution comprising histidine hydrochloride and sucrose;

dissolving doxorubicin hydrochloride in said sucrose - histidine buffer solution to obtain a solution of at least 25mM doxorubicin hydrochloride concentration;

admixing the resultant doxorubicin hydrochloride solution and the extraliposomal ammonium sulphate-freed liposomal composition to obtain a doxorubicin-loaded liposomal composition;

removing extraliposomal doxorubicin hydrochloride from said doxorubicin-loaded liposomal composition by, for example, tangential flow filtration, column chromatography or treatment with resins such as resins based on microporous styrene/divinylbenzene copolymer;

making up the volume of the resultant extraliposomal doxorubicin hydrochloride-freed liposomal composition with a sucrose - histidine buffer solution to obtain a liposomal composition of a desired concentration of doxorubicin;

filtering aseptically, said liposomal composition through a sterile $0.2 \mu m$ sterilising grade filter into a sterile container to obtain said liposomal doxorubicin composition.

Non-pegylated liposomes containing doxorubicin compositions of the present invention have shown decreased toxic effects as compared to conventional doxorubicin hydrochloride formulations (Adriamycin®) and pegylated liposome doxorubicin hydrochloride formulations (Caelyx®). Table 1, below, provides the results of acute toxicity and pharmacokinetic studies in mice. Non-pegylated doxorubicin liposomes of the present invention as manufactured by the parameters set forth in Example II were compared to Adriamycin® and Calyx®. The $LD_{50}$ for the non-pegylated doxorubicin liposomes of the present invention is higher than that for Adriamycin® or Caelyx®, thus demonstrating that the non-pegylated doxorubicin liposomes of the present invention have lower toxicity.

TABLE 1

Acute Toxicity and Pharmacokinetic Studies in Mice

| Parameters | Example II | Caelyx® | Adriamycin® |
|---|---|---|---|
| $LD_{50}$ (mg/kg) | 16.13 | 13.5 | 10.29 |
| MTD (mg/kg) | 8 | 8 | 5 |
| $C_{max}$ ($\mu g/ml$) | 267.54 | 285.74 | 26.8 |
| $T_{max}$ (hours) | 0.085 | 0.085 | 0.085 |
| Kel | 0.0997 | 0.07109 | 4.851811 |
| $T_{1/2}$ (hours) | 6.948 | 9.748 | 0.143 |

(continued)

Acute Toxicity and Pharmacokinetic Studies in Mice

| Parameters | Example II | Caelyx® | Adriamycin® |
|---|---|---|---|
| AUC ($\mu$g -h/ml) | 1694.024 | 2083.215 | 1.244 |
| Vd (ml) | 1.480 | 1.688 | 41.42 |
| Vd (ml/kg) | 59.20 | 67.52 | 1656.79 |
| Cl (ml/h) | 0.15 | 0.12 | 200.96 |

Abbreviations: MTD = maximum tolerated dose; $C_{max}$ = maximum concentration of drug achieved in the plasma; $T_{max}$ = time taken to achieve the maximum concentration of drug in the plasma; Kel = elimination constant; $T_{1/2}$ = time required for the drug concentration in the plasma to get decreased by 50%; AUC = area under "concentration" vs. "time" curve; Vd = volume of distribution; Cl = clearance rate of drug

Non-pegylated doxorubicin liposomes of the present invention were used on MCF-7 human breast tumour implanted in mice. The results are provided in Table 2, below. The difference in tumour weight and effectiveness is measured by T/C% (test to control percentage). In this study (Example VI), the highest ratio of T/C using Caelyx® is -78 at 12mg/kg and -34.7 at 6mg/kg whereas using the non-pegylated doxorubicin liposomes of the present invention, the highest is -93.4 at 12mg/kg and -89.43 at 6mg/kg. These results demonstrate that the non-pegylated doxorubicin liposomal compositions of the present invention appear to be more effective in reducing tumour weight than the currently marketed pegylated liposome formulation, Caelyx®.

TABLE 2:

Effect on Mcf-7 Human Breast Tumour Implanted in Nude Mice Average Tumour Weight (mg)

| Day | Saline Control | Example II (6mg/kg) | Example II (12mg/kg) | Caelyx® (6mg/kg) | Caelyx® (12mg/kg) |
|---|---|---|---|---|---|
| 1 | 36.5 | 31.5 | 68.4 | 38.3 | 57.88 |
| 5 | 36.75 | 45.33 | 81.6 | 44.3 | 50.75 |
| 9 | 63.13 | 40.17 | 43.6 | 41.5 | 31.38 |
| 12 | 52.38 | 42.83 | 46.1 | 60.17 | 32 |
| 16 | 78.13 | 5.33 | 16.2 | 25 | 27.8 |
| 19 | 94 | 3.33 | 8 | 25 | 22.8 |
| 23 | 95.38 | 3.33 | 4.5 | 16 | 16.6 |
| 26 | 94.38 | 3.33 | 4.5 | 25 | 12.6 |
| Wt. gain | 43.4 | -28.17 | -63.9 | -13.3 | -45.2 |
| T/C% | NA | -89.43 | -93.4 | -34.7 | -78 |

Anti-tumour activity of non-pegylated doxorubicin liposomes of the present invention against L1210 mouse leukaemia cells was tested. The results are provided in Table 3, below. The results of this test (Example VI) show that non-pegylated doxorubicin liposomal compositions of the present invention are as effective as the pegylated liposomes (Caelyx®).

TABLE 3:

Anti-Tumour Activity Against L1210 Mouse Leukaemia Model

| Group | Dosage (mg/kg) | Mice | Survival Time (Days) | Mean Survival Time (Days) | T/C% |
|---|---|---|---|---|---|
| Saline Control | NA | 1/5 | 17 | 16 | NA |
| | | 2/5 | 16 | | |
| | | 3/5 | 17 | | |
| | | 4/5 | 16 | | |
| | | 5/5 | 16 | | |

(continued)

Anti-Tumour Activity Against L1210 Mouse Leukaemia Model

| Group | Dosage (mg/kg) | Mice | Survival Time (Days) | Mean Survival Time (Days) | T/C% |
|---|---|---|---|---|---|
| | | 1 /5 | 20 | | |
| | | 2/5 | 20 | | |
| Example II | 6 | 3/5 | 22 | 20.4 | 128 |
| | | 4/5 | 20 | | |
| | | 5/5 | 20 | | |
| | | 1/5 | 23 | | |
| | | 2/5 | 20 | | |
| Example II | 12 | 3/5 | 20 | 21.2 | 132 |
| | | 4/5 | 20 | | |
| | | 5/5 | 23 | | |
| | | 1/5 | 18 | | |
| | | 2/5 | 22 | | |
| Caelyac® | 6 | 3/5 | 20 | 20.4 | 128 |
| | | 4/5 | 20 | | |
| | | 5/5 | 22 | | |
| | | 1/5 | 18 | | |
| | | 2/5 | 22 | | |
| Caelyx® | 12 | 3/5 | 20 | 20.6 | 129 |
| | | 4/5 | 23 | | |
| | | 5/5 | 20 | | |
| T/C %: Test to control percentage | | | | | |

The above results in Tables 1-3 demonstrate that the non-pegylated liposome doxorubicin composition of the present invention has a lower toxicity profile and a longer circulation time than pegylated liposomes and has proven efficacy of anti-tumour activity *in-vivo* efficacy against MCF-7 and L1210 tumour models.

In order that those skilled in the art can more fully understand this invention, the following Examples, which describe the preparation, characterization, and *in vivo* chemotherapeutic application in an animal model of liposome formulations of this invention, are set forth. These Examples are presented solely for purposes of illustration and are not intended to limit the present invention in any way.

Doxorubicin hydrochloride used in these Examples was of parenteral grade complying with US Pharmacopoeial specifications. Phospholipids used in these Examples were of parenteral grade. Cholesterol used in these Examples was complying with US Pharmacopoeial specifications. Water used in these Examples was of parenteral grade complying with Water for Injection specifications. All other additives used in these Examples were of parenteral grade. The entire processing was carried out in an area with a controlled environment.

Caelyx® (pegylated liposomal doxorubicin formulation) manufactured by Ben Venue Laboratories, USA and Adriamycin® (conventional non-liposomal doxorubicin formulation) manufactured by Pharmacia & Upjohn, USA were used in animal studies for comparative evaluation with a non-pegylated liposomal doxorubicin composition of the present invention. Adriamycin® is a freeze-dried sterile powder for injection, each vial containing 10mg doxorubicin hydrochloride, 50mg lactose, and 1mg methylhydroxy-benzoate. Before use, the freeze-dried powder is reconstituted with 5ml of Water for Injection provided with the pack.

For hematological testing, a Cell Counter (Sysmex™ Automated Hematology Analyzer-KX-21) was used.

EXAMPLES

Example I

Process of making a liposomal composition containing doxorubicin.

Lipid film formation:

DSPC (1.565 g) and cholesterol (0.521 g) were dissolved one after the other in chloroform (40ml) in a rotary evaporator flask. They were mixed until a clear solution was formed. The flask was connected to a rotary evaporator and the water bath temperature was adjusted to 60°C. The solvent was evaporated under vacuum to form a thin film of lipids on the wall of the flask. After releasing the vacuum, the flask was rotated for approximately 5 minutes while passing nitrogen into the flask to dry off any residual solvent.

Hydration:

The lipid film in the flask was then hydrated with 60ml of aqueous hydration medium containing ammonium sulphate. The hydration medium consists of 10.0gm of sucrose, 2.04gm of ammonium sulphate, and 100ml of water. The flask containing the lipid film and hydration medium was rotated for 30 minutes on a water bath at a temperature maintained at 65-68°C to form liposomes.

Size reduction of liposomes by extrusion:

The liposomal suspension obtained from above was sized by extruding successively through filters having pore size from 0.4$\mu$m to 0.05$\mu$m.

Development of ammonium sulphate gradient:

The suspension of the sized liposomes was dialyzed against a sucrose-histidine buffer solution to remove extraliposomal ammonium sulphate thereby creating a chemical gradient. A tangential flow filtration system fitted with a 300 KD cassette was used for the dialysis. The absence of ammonium sulphate was tested using Nesseler agent.
The sucrose-histidine buffer solution used in the dialysis and drug loading (below) is as follows: 170.0gm of sucrose, 3.40gm of histidine HCl, 1.7 litres of water, and sodium hydroxide at a quantity sufficient to adjust pH to 6.0 to 6.5.

Drug loading:

In a round bottom flask, a 15mg/ml solution of doxorubicin HCl in sucrose-histidine buffer solution (described above) prepared to load the liposomal preparation and to get drug-loaded liposomes having a concentration of 4mg/ml of doxorubicin (calculated as hydrochloride). The sized and dialyzed liposomes from above were added slowly to the round bottom flask and mixed for one hour at 65°C. The drug-loaded liposomes were mixed with Dowex® for 30 minutes to remove the unentrapped drug. The drug-loaded liposomes were diluted to a 2mg/ml concentration using sucrose-histidine buffer solution and then aseptically filtered using a sterile 0.22$\mu$m membrane filter. The filtered liposomal doxorubicin composition was then filled aseptically into sterile depyrogenated glass vials and sealed under cover of nitrogen using Teflon® coated rubber bungs.

Example II

LD$_{50}$ comparison of doxorubicin formulations.

A liposomal doxorubicin composition was prepared having per ml of the composition having

| DSPC | - | 9.55mg |
| Cholesterol | - | 3.15mg |
| Doxorubicin (as hydrochloride) | | 2.01mg |
| Sucrose | - | 95mg |
| Histidine hydrochloride | - | 2mg |

The composition was prepared by the same procedure as in Example 1.

Doxorubicin hydrochloride (216mg) was dissolved in 14ml of sucrose-histidine buffer solution and added to 40ml of sized liposomes and mixed for 1 hour. The resultant drug loaded liposomal solution dispersion was then passed through a Dowex® column to remove unentrapped drug.

The product obtained after passing through the Dowex® column had the following characteristics:

Product analysis

| | |
|---|---|
| Total doxorubicin content (as HCl) | 3.98mg/ml |
| Entrapped doxorubicin content (as HCl) | 3.94mg/ml |

The above product after dilution with histidine buffer to a concentration of 2mg/ml was analyzed for the following parameters:

| | | |
|---|---|---|
| Appearance | : | Red coloured translucent liquid |
| pH | : | 6.1 |
| Particle size | : | Average particle size 0.093$\mu$m |
| DSPC content | | 9.55mg/ml |
| Cholesterol content | | 3.15mg |
| Doxorubicin content (as HCl) | : | 2.01mg/ml |
| Sucrose content | | 9.35%w/v |
| Histidine HCl content | | Positive |
| Bacterial endotoxins | : | Less than 2.2 EU/mg of doxorubicin hydrochloride. |
| Sterility | : | Sterile |

This composition was subjected to acute toxicity studies in mice.

$LD_{50}$ comparison of doxorubicin formulations.

| | |
|---|---|
| Animals used | : Swiss albino mice of either sex. |
| Weight range of animal | : 20-22gm. |
| Number of groups | : 3 |
| Number of animals per group | : 10 |

Animals were divided into 3 groups and each group comprised of ten animals. Group 1 received a composition of Example II, Group 2 received Caelyx®, and Group 3 received Adriamycin®.

All animals received injections via the intravenous route. The drug solutions were suitably diluted with dextrose (5%w/v) solution before administering to the animals. The animals were then observed for a period of 14 days. They were observed for any clinical toxicity and mortality.

$LD_{50}$ values of different Doxorubicin formulations studied are provided in Table 1.

$LD_{50}$ dose was found to be 16.13mg/kg whereas the $LD_{50}$ dose for the marketed conventional preparation (Adriamycin®) was 10.29mg/kg. The $LD_{50}$ for the marketed pegylated liposomal preparation Caelyx® was 13.5mg/kg. These results show that that non-pegylated liposomes of the present invention have a reduced toxicity as compared to other doxorubicin formulations and to pegylated-liposomal doxorubicin formulations, including pegylated-liposomal doxorubicin formulations.

Example III

Comparison of Subacute Toxicity of Doxorubicin Formulations.

| Animals used | : Swiss albino mice of either sex |
|---|---|
| Number of groups | : 11 |
| Number of animals per group | : 8 |
| Weight range of animal | : 19-23gm |
| Route of administration | : Intravenous |

Animals were divided into 11 groups, each group comprising of eight animals. Group 1 received Dextrose 5% Injection, Group 2 received blank liposomes (before drug loading) of the present invention, Group 3, Group 4 and Group 5 received a composition of Example II at different doses, Group 6, Group 7 and Group 8 received Caelyx® at different doses, Group 9, Group 10 and Group 11 received Adriamycin® at different doses. The doses are provided in Table 4.

TABLE 4

Doses of Doxorubicin Formulations for Repeat Dose Toxicity Studies in Mice

| Group No. | Group | Dose | Cumulative dose |
|---|---|---|---|
| | | (mg/kg body weight) | (mg/kg body weight) |
| 1 | Dextrose | - | - |
| 2 | Blank liposomes | - | - |
| 3 | | 1 | 7 |
| 4 | Example II | 2 | 14 |
| 5 | | 3 | 21 |
| 6 | | 1 | 7 |
| 7 | Caelyx® | 2 | 14 |
| 8 | | 3 | 21 |
| 9 | | 1 | 7 |
| 10 | Adriamycin® | 2 | 14 |
| 11 | | 3 | 21 |

All groups received injections on alternate days, for fourteen days via the intravenous route. The formulations were suitably diluted with Dextrose 5% Injection before administration to the animals. The animals were observed during the study period of 14 days for the following:

mortality,
clinical signs and symptoms,
body weights,
food consumption and
organ weights.

RESULTS

Mortality:

The percent mortality over a period of fourteen days was recorded for all the formulations.

TABLE 5

Percent Mortality for the Various Doses of Doxorubicin Formulations

| Group | Dose | Percent Mortality |
|---|---|---|
| | (Mg/Kg Body Weight) | |
| Dextrose | - | 0 |
| Blank liposomes | - | 0 |
| | 1 | 0 |
| Example II | 2 | 0 |
| | 3 | 0 |

(continued)

Percent Mortality for the Various Doses of Doxorubicin Formulations

| Group | Dose | Percent Mortality |
|---|---|---|
| | (Mg/Kg Body Weight) | |
| | 1 | 0 |
| Caelyx® | 2 | 0 |
| | 3 | 0 |
| | 1 | 0 |
| Adriamycin® | 2 | 0 |
| | 3 | 12.5 |

Clinical signs:

During the course of study, shedding of tail skin and alopecia was observed in all doxorubicin treated groups. Shedding of tail skin was observed in animals after five injections. Dose dependent alopecia was observed in all of the doxorubicin treated animals. Table 6 details the alopecia during the course of this study.

TABLE 6

Incidence of Alopecia in Mice Treated with Various Doxorubicin Formulations

| Formulation | Grading of alopecia |
|---|---|
| Dextrose | - |
| Blank Liposomes | - |
| Example II (1 mg/kg) | + |
| Example II (2mg/kg) | + |
| Example II (3mg/kg) | + + |
| Caelyx® (1 mg/kg) | Piloerection # |
| Caelyx® (2mg/kg) | + |
| Caelyx® (3mg/kg) | + + |
| Adriamycin® (1mg/kg) | + |
| Adriamycin® (2mg/kg) | + + |
| Adriamycin® (3mg/kg) | + + + + |

# Piloerection (raising of hair) was observed in one out of 8 animals on day 12 of the treatment.
+ One out of 8 animals showed alopecia
+ + Two out of 8 animals showed alopecia
+ + + Three out of 8 animals showed alopecia
+ + + + Four out of 8 animals showed alopecia

Body weight:

The body weights of animals were recorded on day 1, day 4, day 7 and day 14. At the dose of 2mg/kg and 3mg/kg, a decrease in the body weights was observed in all drug treated groups. The weight loss was significantly different from the control. The body weight of animals receiving blank liposomes was comparable to the dextrose group.

Food Consumption:

From a period of 4 to 14 days doxorubicin treated animals showed in general a decrease in food consumption.

Organ weights:

The organs of surviving animals were collected and weighed. The mean organ weights of all the animals were found to be comparable in all drug treated groups.

Example IV

Evaluation of pharmacokinetic of doxorubicin formulations.

| | |
|---|---|
| Animals used | : Swiss albino mice of either sex |
| Number of groups | : 3 |
| Number of animals per group | : 48 |
| Animal body weight | : 25 -30gm |
| Dose for pharmacokinetic study | : 10mg/kg |
| Time points | : 5min, 30 min, 1h, 2h, |
| 5h, 0h, 15h,20h | |
| Number of mice per time point | : 6 mice |

Blood samples after collection were centrifuged at 4000 rpm for 20 min and the plasma was separated and frozen at -20˚C until analysed. The frozen plasma was thawed and used for analysis.

1ml of acetonitrile was added to 100 $\mu$L of plasma, vortexed for 10 min, centrifuged at 3250 rpm for 10 min. The supernatant was withdrawn and 0.5ml of saturated $ZnSO_4$ solution was added to it. The resulting solution was vortexed for 5 min and then centrifuged for 10 min at 3250 rpm. The upper organic layer was then withdrawn and dried under oxygen free nitrogen gas at 60˚C. The residue obtained was then reconstituted with 200$\mu$L of Solvent A containing $ZnSO_4$. 100 $\mu$L of this solution was then injected in the HPLC column.

| | |
|---|---|
| Instrument | Shimadzu Liquid Chromatograph LC-10AT$_{VP}$ |
| Column | C8 Thennoquest™ hypersil MOS (250 X 4.6mm, 5$\mu$) |
| Column Temp | Ambient |
| Mobile Phase | Solvent A :Acidified Water (pH 2.5, adjusted with 60% perchloric acid) & tetrahydrofuran (80:1, v/v) |
| | Solvent B : Acetonitrile |
| | Solvent A:Solvent B (40:60) |
| Flow Rate | 1ml/min |
| Detector | Fluorescent Detector (RF - 10 AXL Shimadzu; Ex 460nm and Em 550nm |
| Run time | 15 min |

Statistical analysis

Student's t-test was used for comparison between the three formulations. The results are summarized in Table 1

Example V

Comparison of Subacute Toxicity of Doxorubicin Formulations.

| | |
|---|---|
| Animals used | : Dogs |
| Number of groups | : 3 |
| Number of animals per group | : 3 |
| Weight range of animal | : 10-20 kg |
| Dosage & administration | : 1 mg/kg by Intravenous infusion over 20 minutes. Administration was done once a week (i.e. after 7 days) for 4 doses. |

Pharmacological evaluation

clinical signs of toxicity,
body weight,
haemodynamic parameters,
haematology and
biochemical parameters

TABLE 7

| Clinical Signs of Toxicity: | | | |
|---|---|---|---|
| Signs | Control (Dextrose 5% Injection) | Adriamycin® | Example II |
| Dermal lesion | None of signs were seen in this group | Alopecic lesions, erythemic lesions seen after third dose | None of signs were seen in this group |
| Vomiting | | At first and second dose - 2/3 Third and fourth dose - 1/3 | |
| Diarrhoea | | 1/3 at after second, third and fourth dose | |
| Others | | Anorexia | |

Body weight:

The Adriamycin® treated group showed decrease in the bodyweight whereas the control and composition of Example II treated groups showed no change in body weight.

TABLE 8

| Haemodynamic Parameters: | | | |
|---|---|---|---|
| Parameters | Control (Dextrose 5% Injection) | Adriamycin® | Example II |
| Blood pressure | Normal | Normal | Normal |
| Heart rate | Normal | Increases by average + 29.17% | Normal |
| Respiratory rate | Normal | Decreases by average -42.12% | Normal |
| Temperature | Increases body temperature during and after administration (clinically non-significant) | | |

Haematological parameters studied:

RBC,
total WBC and differential WBC,
haemoglobin,
haematocrit,
mean corpuscular volume, and
platelet.

All the above parameters studied were within normal range in all the groups.

Biochemical parameters:

Increase in creatinine phosphokinase and lactate dehydrogenase levels were found in the Adriamycin® treated group whereas in control and the composition of Example II groups there was no significant change observed.

Liver Function Test (LFT):

Increase in aspartate aminotranferase, alanine aminotranferase and total bilirubin levels were observed in the Adriamycin® treated group whereas in control and the composition of Example II groups no significant changes were observed.

Kidney Function Test (KFT):

Increase in blood urea nitrogen (BUN) and creatinine were observed in the Adriamycin® treated group whereas the control group showed no increase. The group treated with composition of Example II showed an increase in both BUN and creatinine levels which however were significant less than the Adriamycin® treated group.

Example VI

Evaluation of the Anti-tumour Activity of non-pegylated liposome doxorubicin formulation of the present invention with pegylated liposome doxorubicin formulation (Caelyx®) against L1210 mouse leukaemia and MCF-7 human breast tumour implanted in nude mice.

Dose Preparation:

Both the above doxorubicin formulations were diluted to 1mg/ml with sterile normal saline (0.9%). Appropriate volumes of drug solution was administered to various test groups on the basis of body weight so that the animals received the drug as indicated in Tables 9 and 10.

Six-week old female NCr nude (nu/nu) mice were used in both models.

The animals were housed in polycarbonate microisolator cages as specified in the Guide for Care and Use of Laboratory Animals (ILAR publication, 1996, National Academy Press). The rooms were well ventilated (greater than 10 air changes per hour) with 50% fresh air. A 12-hour light/12-hour dark photoperiod was maintained. The room temperature was maintained between 18-26°C.

The study animals were acclimatized for at least 3 days prior to tumour inoculation.

General Description:

Both liposomal formulations listed above were tested in L1210 mouse leukaemia and MCF-7 human breast tumour models at two concentrations each against a control group receiving saline.

L1210 Model

Tumour Cells:

L1210 mouse leukaemia cell line was obtained from ATCC and propagated using standard *in vitro* cell expansion methods. The cells were grown in culture medium with appropriate supplements and 10% Foetal bovine serum (FBS). The culture was then grown in 35 T-225 flasks to 80-90% confluence. The cells were harvested by centrifugation and the pelleted cells were resuspended in serum-free RPMI to $10^6$ viable cells/ml. The animals were injected with 0.1ml of cell suspension using a 25G needle.

Groups and Dosages:

Each group consisted of 5 animals. Mice were inoculated intraperitoneally with $10^6$ tumour cells/mouse. Both the liposomal formulations were administered intravenously on day 1, 5 and 9 at dosages shown in Table 9. The animals were observed for 30 days post treatment and mortality was recorded.

TABLE 9

| Group No. | Number of Males/ Females | Formulation | Total Dose (mg/kg) | Dose/injection (mg/kg) | Total number of doses |
|---|---|---|---|---|---|
| 1 | 0/5 | Saline | NA | NA | 3 |
| 2 | 0/5 | Caelyx® | 12 | 4 | 3 |
| 3 | 0/5 | Caelyx® | 6 | 2 | 3 |
| 4 | 0/5 | Example II | 12 | 4 | 3 |
| 5 | 0/5 | Example II | 6 | 2 | 3 |

The animals were examined daily and weighed twice every week and the weights were recorded. Any mortality during the course of the study was recorded.

The anti-tumour activities of both the liposomal formulations were evaluated by comparing the mean survival time in each treated group to that of the controls which received saline. The results were expressed in terms of T/C ratios which was calculated as follows:

$$T/C\% = \frac{\text{Mean survival time of test group}}{\text{Mean survival time of control group}} \times 100$$

A T/C ≤125% is considered significant activity

The results of anti-tumour activity against L1210 Mouse leukaemia model are provided in Table 3.

Mortalities ranged from 15 to 26 days after the first injection (Day 1). The mean survival time of the control group, which received saline was 16.5 days. Increase in the survival time was observed in both the drug treated groups. Both the drug treated groups showed similar difference in the mean survival time (T/C%) indicating that the composition of Example II is as efficacious as Caelyx® against L1210 tumour model.

MCF-7 Model:

Tumour Cells:

MCF-7 human breast tumour cell line was obtained from ATCC and propagated using standard *in vitro* cell expansion methods. The cells were grown in culture medium with appropriate supplements and 10% FBS. The culture was then grown in 35 T-225 flasks to 80-90% confluence. The cells were harvested by centrifugation and the pelleted cells were trypinized and resuspended in serum-free RPMI to $10^7$ viable cells/ml. The animals were injected with 0.1ml of cell suspension using a 25G needle.

Groups and Dosages:

Each group consisted of 5 animals. Mice were implanted with estrogen pellets 5 days prior to inoculation. They were inoculated subcutaneously with $10^7$ tumour cells/mouse. The tumour was allowed to grow until they reach a size of 30-100 mm³. Once appropriate size has been reached (5th day after inoculation), mice were be dosed intravenously with the test formulation on day 1, 5 and 9 as shown in Table 10. Tumour size was measured using a calliper twice weekly up to 30 days post treatment initiation.

TABLE 10

| Group No. | Number of Males/Females | Formulation | Total Dose (mg/kg) | Dose/injection (mg/kg) | Total number of doses |
|---|---|---|---|---|---|
| 1 | 0/5 | Saline | - | - | - |
| 2 | 0/5 | Caelyx® | 12 | 4 | 3 |
| 3 | 0/5 | Caelyx® | 6 | 2 | 3 |
| 4 | 0/5 | Example II | 12 | 4 | 3 |
| 5 | 0/5 | Example II | 6 | 2 | 3 |

The animals were examined daily and weighed twice every week and the weights were recorded. The length and the width for tumours of individual mice was measured twice a week using callipers and the approximate tumour weight (mg) from tumour dimensions (mm X mm) was calculated using the formula for volume of a prolate ellipsoid:

$$\frac{L \times W^2}{2}$$

where L is the longer of the two measurements.

The anti-tumour activity of both the liposomal formulations were evaluated by comparing the change in tumour weight for treated group to that of the controls, which received saline.

The change in tumour weight was calculated by subtracting the group median tumour weight on day 5 post-inoculation of tumour cells from group median tumour weight on the final evaluation day (day 30 post-treatment).

$$\upsilon\ Wt\ =\ Wt_{final}\ --\ Wt_{initial}$$

The T/C ratio for all test groups was calculated as follows:

$$T/C\%\ =\ \upsilon\ Wt\ Test\ /\ \ Wt_{initial}\ of\ Test\ X\ 100$$

A T/C ≤20% is considered necessary to demonstrate moderate activity. A T/C ≤10% is considered significant activity.

The anti-tumour activity against MCF-7 human breast tumour model is tabulated in Table 2.

TABLE 11
Early Deaths in Various Groups of Animals

| Group | Dosage | Mortality |
|---|---|---|
| Control | Nil | 0/5 |
| Example II | 6mg/kg | 0/5 |
| Example II | 12mg/kg | 0/5 |
| Caelyx® | 6mg/kg | 2/5 |
| Caelyx® | 12mg/kg | 1/5 |

Tumours in the control group continued to grow throughout duration of the study reaching a maximum of 116.4mg on the 26th day whereas tumours in the treated mice regressed significantly during the course of the study. The tumours disappeared completely in the group receiving 12mg/kg of composition of Example II formulation indicating that composition of Example II is effective against MCF-7 human breast tumours.

Several early deaths occurred in various groups as shown in Table 11. However, the cause of deaths seemed to be unrelated to the tumours. There were no deaths in saline control group, which had the largest tumours. Some of the dead animals were necropsied, and all of them were found to have thickened, abnormal bladders. At the termination of the study, many of the euthanized mice, likewise had thickened bladders. Histopathological examination of one of the thickened bladders revealed no evidence of tumour metastasis. Premature death of estrogenised, tumour-implanted nude mice due to the incidence of urogenital disease.

Example VII

Determination of Maximum Tolerated Dose (MTD) and to assess therapeutic efficacy of doxorubicin liposomes of the present invention in nude athymic mice with A121 human ovarian tumour.

Maximum tolerated dose and assessment of therapeutic efficacy of doxorubicin liposomes of the present invention in nude athymic mice with A121 human ovarian tumour was carried out in comparison with a conventional non-liposomal formulation (Adriamycin®) and a pegylated liposomal formulation (Caelyx®).

Nude athymic Ncr-nu/nu mice [4 mice/group (10 in Control group)] were implanted subcutaneously with human A121 ovarian tumour via trocar implant. A total for 46 animals were used in this experiment. A total of 46 animals were utilised for the experiment. Equivalent doses of Adriamycin®, Caelyx® and the composition of Example II were evaluated intravenously. Drugs were administered intravenously via tail vein of mice on day 5 and 12 after tumour implant.

All treatment groups demonstrated good antitumour efficacy.

The dosage schedule is presented below.

Control

The control mice received no treatment.

Adriamycin®

12mg/kg (6mg/kg x 2 injection)
24mg/kg (12mg/kg x 2 injection)
36mg/kg (18mg/kg x 2 injection)

Caelyx®

12mg/kg (6mg/kg x 2 injection)
24mg/kg (12mg/kg x 2 injection)
36mg/kg (18mg/kg x 2 injection)

Composition of Example II

12mg/kg (6mg/kg x 2 injection)
24mg/kg (12mg/kg x 2 injection)
36mg/kg (18mg/kg x 2 injection)

All mice receiving the highest dosage 36mg/kg of free drug (18mg/kg x 2 Adriamycin®) and 3 of 4 mice that received the intermediate dosage of 24mg/kg died as a result of drug toxicity. The maximum tolerated dose (MTD) of Adriamycin® is hence less than 24mg/kg.

Mice tolerated both Caelyx® and the composition of Example II. Both the formulations were well tolerated at 36mg/kg. However, Caelyx® appeared to cause more toxicity than the composition of Example II and produced a greater weight loss of mice receiving the high dose (36mg/kg).

This study demonstrates that the composition of Example II is better tolerated than the commercially available pegylated liposomal preparation (Caelyx®) and conventional non-liposomal formulation (Adriamycin®).

Example VIII

To assess the efficacy of liposomal doxorubicin composition of the present invention in nude athymic mice implanted with a multidrug resistant, Pgp positive, human colon DLD1 I tumour xenografts.

The composition of Example II along with Calyx® and Adriamycin® were subjected to efficacy studies in nude athymic mice implanted s.c. with the drug resistant (Pgp+) DLD-1 human colon tumour.

Animals, nude athymic mice, 4 mice/group (10 in Control group) implanted subcutaneously with human DLD-1 colon tumour via trocar implant.

Control

The control mice received no treatment.

Adriamycin®

12mg/kg (6mg/kg x 2 injection)
24mg/kg (12mg/kg x 2 injection)

Caelyx®

24mg/kg (12mg/kg x 2 injection)
36mg/kg (18mg/kg x 2 injection)
48mg/kg (24mg/kg x 2 injection)

Composition of Example II

24mg/kg (12mg/kg x 2 injection)
36mg/kg (18mg/kg x 2 injection)
48mg/kg (24mg/kg x 2 injection)

A total of 42 animals were utilised for the experiment.

Results:

The dosages of Adriamycin® were lowered to 12 and 24 mg/kg in this study based on the toxicity observed in Example VII following the administration of 36mg/kg free drug. In contrast, dosages of Caelyx® and the composition of Example II were increased to 48mg/kg to compare their efficacies and toxicities with the free drug at their respective MTDs. All agents were administered to nude athymic mice i.v. via tail vein on day 5 and 12 after s.c. tumour implant with the multidrug resistant, Pgp positive, human colon tumour xenograft.

All treatments groups demonstrated antitumour efficacy. However, mice receiving either of the liposomal preparations demonstrated significantly greater antitumour efficacy. At equivalent free drug dosages (24mg/kg), a median tumour growth delay of 10 days was observed with the free drug, while all mice administered liposomal preparations had tumours that were less than $600nm^3$ on day 40. No toxicity was evident at dosages of 36mg/kg for either Caelyx® or composition of Example II.

At the highest dosages (48mg/kg) both liposomal drug formulations (24mg/kg x 2, Caelyx® or composition of Example II), mice demonstrated >15% weight loss, and 1 of 4 animals of each of those groups died early (day 17, 19) as a result of drug toxicity. Therefore, the MTD of the both liposomal formulations was similar and appeared to be less than 48mg/kg.

In contrast to Adriamycin®, the two liposomal formulations [Caelyx®-(pegylated doxorubicin) and composition of Example II (non-pegylated-doxorubicin)] displayed significant antitumour efficacy against s.c. implanted, Pgp positive, multidrug resistant human DLD1 colon tumours in nude athymic mice. At equivalent dosages of 24mg/kg, both liposomal formulations displayed increased efficacy as compared with the free drug. In addition, both liposomal formulations displayed lower toxicities as compared with the free drug allowing more drug to be administered. The MTD for Adriamycin® appears to be about half that of the liposomal formulations. Liposomal drug dosages of 36mg/kg were well tolerated.

Examples IX to XIII

The composition and process of Example IX to XIII are given in Table 12.

TABLE 12

| | Example IX | Example X | Example XI | Example XII | Example XIII |
|---|---|---|---|---|---|
| Parameters changed → <br><br> Ingredients ↓ | Increased Particle size | Less Cholesterol | Higher cholesterol | $C_{14}$ phospholipid | Conventional hydration |
| DSPC | 1.565g | 1.565g | 1.565g | - | 1.565g |
| DMPC | - | - | - | 1.565g | - |
| Cholesterol | 0.521 g | 0.3 g | 0.74 g | 0.521 g | 0.521 g |
| Chloroform | 40ml | 40ml | 40ml | 40ml | 40ml |
| Hydrating medium | 60ml | 60ml | 60ml | 60ml | 120ml |
| Average particle Size | 0.18μm | 0.085μm | 0.095μm | 0.095μm | 0.085μm |

(continued)

|  | Example IX | Example X | Example XI | Example XII | Example XIII |
|---|---|---|---|---|---|
| Histidine Buffer | 1.7 1 | 1.71 | 1.7 | 1.7 | 1.71 |
| Doxorubicin HCl | 330mg | 330mg | 330mg | 330mg | 330mg |
| Histidine buffer (for solubilizing the drug) | 22ml | 22ml | 22ml | 22ml | 40ml |
| Histidine buffer (for dilution) | 80ml | 80ml | 80ml | 80ml | - |

Procedure:

Procedure of Example I was followed for Examples X, XI and XII. In Example IX, the procedure of Example I was followed except for the size reduction of liposomes which was carried out by extruding through membranes of 0.4μm to 0.2μm to get an average size in the range of 0.15μm to 0.25μm. In Example XIII, the procedure of Example II was followed except for the volume of hydration which was doubled.

The results of toxicological testing are given in Table 13.

TABLE 13

| Observations | | | | | |
|---|---|---|---|---|---|
|  | Example IX | Example X | Example XI | Example XII | Example XIII |
| Parameters changed → | Increased Particle size | Less Cholesterol | Higher cholesterol | C14 phospholipid | Conventional hydration |
| Results ↓ | | | | | |
| $T_{1/2}$ in mice | 2 h ($C_{max}$ and AUC not comparable) | 3 h | 5 h ($C_{max}$ and AUC not comparable) | 2 h | 4 h |
| $LD_{50}$ in mice | 12mg/kg | 10mg/kg | 12mg/kg | 10mg/kg | 14mg/kg |
| Conclusion (with reference to composition of Example I) | $T_{1/2}$ significantly less | $T_{1/2}$ significantly less and increased toxicity | $C_{max}$ and AUC were significantly less | $T_{1/2}$, $C_{max}$, AUC significantly less | Less $T_{1/2}$ |

Example XIV (Comparative)

Liposomal doxorubicin composition without sucrose.

Lipid film formation:

Distearoylphosphatidylcholine (1.565 g) and cholesterol (0.521 g) were dissolved one after the other in chloroform (40ml) in a rotary evaporator flask. They were mixed until a clear solution was formed. The flask was connected to a Rotary evaporator and the water bath temperature was adjusted to 60˚C. The solvent was evaporated under vacuum to form a thin film of lipids on the wall of the flask. After releasing the vacuum, the flask was rotated for approximately 5 minutes while passing nitrogen into the flask to drive off any residual solvent.

Hydration:

The lipid film was hydrated with 60ml of aqueous hydration medium. The aqueous hydration medium was 2.04%w/v ammonium sulphate in water. The flask containing the lipid film and hydration medium was rotated for 30 minutes on a water bath maintained at 65 - 68˚C to form blank liposomes.

Size reduction of blank liposomes by extrusion:

The liposomal suspension obtained from above was sized by extruding successively through filters having pore size from 0.4μm and to 0.05μm.

Dialysis:

The suspension of the sized liposomes was dialyzed against a 0.2%w/v histidine hydrochloride solution of pH 6.5. A tangential flow filtration system was used for the dialysis. The dialysis was continued until extra liposomal ammonium sulphate was removed. The absence of ammonium sulphate in extra liposomal media was confirmed using Nesseler reagent.

Drug loading:

In a round bottom flask, a 15mg/ml solution of doxorubicin HCl was prepared by dissolving 216mg of doxorubicin hydrochloride in 14ml of histidine hydrochloride solution (described above). The measured volume (40ml) of sized and dialyzed liposomes from above were added slowly to the round bottom flask and mixed for one hour at 65˚C.

The drug-loaded liposomes were treated with Dowex® to remove the unentrapped drug.

The samples of the composition obtained before and after treatment with Dowex® were analysed for doxorubicin content by high pressure liquid chromatography (HPLC). The results are as follows:

Total doxorubicin content (as HCl) (before Dowex® treatment)    4.02mg/ml
Entrapped doxorubicin content (as HCl) (after Dowex® treatment)    4mg/ml

The doxorubicin loaded liposomes after removing the free drug were diluted to a 2mg/ml of doxorubicin (as hydrochloride) concentration using a solution of histidine hydrochloride and sucrose (described above). The liposomal composition thus obtained was then aseptically filtered using a sterile 0.22μm membrane filter into a sterile depyrogenated container and was analyzed for the following parameters:

Appearance            : Red coloured translucent liquid
pH                    : 6.3
Particle size         : Average particle size 0.097μm
Doxorubicin HCl content : 2.05mg/ml
Bacterial endotoxins  : Less than 2.2 EU/mg of doxorubicin (as hydrochloride).
Sterility             : Sterile

Stability studies on the composition obtained in this Example were carried out and the observations are given in Table 14.

Example XV:

Process of making a liposomal doxorubicin composition with 120mM ammonium sulphate solution. (Comparative).

Lipid film formation:

Distearoylphosphatidylcholine (1.565 g) and cholesterol (0.521 g) were dissolved one after the other in chloroform (40ml) in a rotary evaporator flask. They were mixed until a clear solution was formed. The flask was connected to a rotary evaporator and the water bath temperature was adjusted to 60˚C. The solvent was evaporated under reduced pressure to form thin film of lipids on the wall of the flask. After releasing the vacuum, the flask was rotated for approximately 5 minutes while passing nitrogen into the flask to drive off any residual solvent.

Hydration:

The lipid film was hydrated with 60ml of aqueous hydration medium. The aqueous hydration medium consists of sucrose 10%w/v, ammonium sulphate 1.58%w/v in water. The flask containing the lipid film and hydration medium was rotated for 30 minutes on a water bath maintained at 65 - 68°C to form blank liposomes.

Size reduction of blank liposomes by extrusion:

The liposomal suspension obtained from above was sized by extruding successively through filters having pore size from 0.4$\mu$m to 0.05$\mu$m.

Dialysis:

The suspension of the sized liposomes was dialyzed against a histidine buffer. A tangential flow filtration system was used for the dialysis. The dialysis was continued till extraliposomal ammonium sulphate was removed. The absence of ammonium sulphate in extraliposomal media was confirmed using Nesseler reagent. The histidine hydrochloride solution used in the dialysis and drug loading (below) was as follows: 170.0gm of sucrose, 3.40gm of histidine HCl, 1.7 litres of water, and sodium hydroxide at a quantity sufficient to adjust pH to 6.0 to 6.5.

Drug loading:

In a round bottom flask, a 15mg/ml solution of doxorubicin HCl was prepared by dissolving 216mg of doxorubicin hydrochloride in 14ml of histidine hydrochloride solution (described above). The measured volume (40ml) of sized and dialyzed liposomes from above were added slowly to the round bottom flask and mixed for one hour at 65°C.
The drug-loaded liposomes were treated with Dowex® to remove the unentrapped drug.
The samples of the composition obtained before and after treatment with Dowex® were analysed for doxorubicin content by high pressure liquid chromatography (HPLC). The results are as follows:

> Total Doxorubicin content (as HCl) (before Dowex® treatment)    4.11mg/ml
> Entrapped Doxorubicin content (as HCl) (after Dowex® treatment)    4.10mg/ml

The doxorubicin loaded liposomes after removing the free drug were diluted to a 2mg/ml of doxorubicin (as hydrochloride) concentration using a solution of histidine hydrochloride and sucrose (described above). The liposomal composition thus obtained was then aseptically filtered using a sterile 0.22$\mu$m membrane filter into a sterile depyrogenated container and was analyzed for the following parameters:

> Appearance          : Red coloured translucent liquid
> pH                  : 6.35
> Particle size       : Average particle size 0.09$\mu$m
> Doxorubicin HCl content  : 2.03mg/ml
> Bacterial endotoxins : Less than 2.2 EU/mg of doxorubicin (as hydrochloride).
> Sterility           : Sterile

Stability studies on the composition obtained in this Example were carried out and the observations are given in Table 14.

Example XVI:

Composition of Example XIV (Comparative), Example XV along with the Composition of present invention (Example II) were subjected for short-term stability studies at accelerated temperature (25°C).

Results of doxorubicin content are given in Table 14 :

TABLE 14

| | Example II (Hydrated with sucrose and 155mM ammonium sulphate solution) | | Example XIV (Comparative) (Hydrated with 155mM ammonium sulphate solution without sucrose) | | Example XV (Comparative) (Hydrated with sucrose and 120mM ammonium sulphate solution) | |
|---|---|---|---|---|---|---|
| | Entrapped (mg/ml) | Total (mg/ml) | Entrapped (mg/ml) | Total (mg/ml) | Entrapped (mg/ml) | Total (mg/ml) |
| Initial | 2.01 | 2.01 | 2.05 | 2.05 | 2.03 | 2.03 |
| 25°C-1 week | 2.01 | 2.01 | 1.86 | 2.04 | 1.84 | 2.03 |

This Example shows that presence of sucrose is essential for reducing leakage of encapsulated doxorubicin and ammonium sulphate concentration in hydration medium is important. A concentration of 120mM leads to leakage of encapsulated doxorubicin and hence not satisfactory. However, the composition of Example II containing sucrose and ammonium sulphate in a concentration of 155mM did not leak the encapsulated doxorubicin during the study duration.

Example XVII:

Preparation of liposomal doxorubicin composition by the process of solvent removal after hydration.

Distearoylphosphatidylcholine (1.565 g) and cholesterol (0.521 g) were dissolved one after the other in ethanol (20ml) and pumped slowly under pressure into the aqueous hydration medium which was constantly stirred. The aqueous hydration medium consisted of sucrose 10%w/v, ammonium sulphate 2.04%w/v in water. This lipid solution containing the solvent ethanol was transferred to rotary evaporator flask. Flask was connected to a rotary evaporator and the water bath temperature was adjusted to 60°C. Ethanol was removed under vacuum.

Size reduction of blank liposomes by extrusion:

The liposomal suspension obtained from above was sized by extruding successively through filters having pore size from 0.4μm and to 0.05μm.

Dialysis:

The suspension of the sized liposomes was dialyzed against a histidine buffer. A tangential flow filtration system was used for the dialysis. The dialysis was continued until extra liposomal ammonium sulphate was removed. The absence of ammonium sulphate in extra liposomal media was confirmed using Nesseler reagent. The histidine hydrochloride solution used in the dialysis and drug loading (below) was as follows: 170.0gm of sucrose, 3.40gm of histidine HCl, 1.7 litres of water, and sodium hydroxide at a quantity sufficient to adjust pH to 6.0 to 6.5.

Drug loading:

In a round bottom flask, a 15mg/ml solution of doxorubicin HCl was prepared by dissolving 216mg of doxorubicin hydrochloride in 14ml of histidine hydrochloride solution (described above). The measured volume (40ml) of sized and dialyzed liposomes from above were added slowly to the round bottom flask and mixed for one hour at 65°C.

The drug-loaded liposomes were treated with Dowex® to remove the unentrapped drug.

The doxorubicin loaded liposomes after removing the free drug were diluted to a 2mg/ml of doxorubicin (as hydrochloride) concentration using solution of histidine hydrochloride and sucrose (described above). The liposomal composition thus obtained was then aseptically filtered using a sterile 0.22μm membrane filter into a sterile depyrogenated container.

Summary of the toxicological and efficacy studies carried out are as follows:

Example II - Non-pegylated long circulating liposomes containing doxorubicin of the present invention have shown decreased toxic effects as compared to non-liposomal doxorubicin hydrochloride formulations (Adriamycin®) and pegylated liposomal doxorubicin hydrochloride formulations (Caelyx®). The $LD_{50}$ for the non-pegylated doxorubicin liposomes of the present invention is higher than the Caelyx® and Adriamycin®, thus demonstrating that the non-pegylated doxorubicin liposomes of the present invention have lower toxicity.

Example III - In sub-acute toxicity study, similar pattern of toxicity was observed in both the Caelyx® and composition of Example II groups whereas Adriamycin® showed toxicity.

Example IV - In pharmacokinetic study, composition of Example II and Caelyx® showed comparable plasma half-life. The apparent volume of distribution is approximately equal to the total blood volume which indicated low liposomal uptake by normal tissues and was similar to Caelyx®. Adriamycin® showed faster clearance rate and high volume of distribution indicating uptake of free doxorubicin in normal tissues.

Example V - In dog toxicity study, composition of Example II found to be better tolerated than Adriamycin®.

Example VI - In tumour models of L1210 mouse leukaemia and MCF-7 human breast tumour, composition of Example II was found to be efficacious.

Example VII - Maximum tolerated dose of the composition of Example II was found to be much higher than Adriamycin® in tumour implanted mice.

Example VIII - Composition of Example II was found to be efficacious in nude athymic mice implanted with multidrug resistant, Pgp positive, human colon DLD1 tumour xenografts.

The above Examples clearly prove that the compositions of the present invention are very useful for reducing tumour growth. This involves parenterally administering a therapeutically effective amount of non-pegylated doxorubicin liposomes of the present invention. The non-pegylated doxorubicin liposomes have a prolonged circulation time, exhibit decreased toxicity and do not present "Hand-Foot Syndrome" issues and hence they are useful for reducing tumour growth.

**Claims**

1. A process for manufacture of long circulating non-pegylated liposomes comprising;

    dissolving one or more phospholipids and one or more sterols in a solvent or mixture of solvents;
    hydrating the resultant lipids; and
    removing said solvent(s) before or after said hydration,

    wherein said hydration is with an aqueous hydration medium in an amount in the range of 10 to 35 ml for each mmole of phospholipid present in the lipid solution to form non-pegylated liposomes, **characterized in that** the aqueous hydration medium comprises sucrose and not less than 125mmoles/litre ammonium sulphate.

2. A process of Claim 1, wherein the sucrose concentration in the aqueous hydration medium is from 0.1M to 0.5M.

3. A process of Claim 2, wherein the sucrose concentration is from 0.25M to 0.3M.

4. A process of Claim 3, wherein the sucrose concentration is about 0.27M.

5. A process of any one of the preceding claims, wherein the liposomes in the liposomal composition are sized to $0.06\mu m$ to $0.16\mu m$.

6. A process of any one of the preceding claims, wherein extraliposomal hydration salt is removed from the liposomal composition using a dialysis buffer solution.

7. A process of any one of the preceding claims, wherein the amount of aqueous hydration medium used is 20 to 35 ml for each mmole of phospholipid in the lipid solution.

8. A process of Claim 7, wherein the amount of aqueous hydration medium used is about 30ml for each mmole of phospholipid in the lipid solution.

9. A process of any one of the preceding claims, wherein the molar ratio of phospholipid to sterol is from 1:0.1 - 1:2.

10. A process of Claim 9, wherein the wherein the molar ratio of phospholipid to sterol is about 1:0.7.

11. A process of any one of the preceding claims, wherein the phospholipid is selected from distearoyl phosphatidylcholine (DSPC), dipalmitoyl phosphatidylcholine (DPPC), hydrogenated soya phosphatidylcholine (HSPC), and derivatives of such phospho lipids.

12. A process of any one of the preceding claims, wherein the phospholipid is distearoyl phosphatidylcholine (DSPC).

13. A process of any one of the preceding claims, wherein the sterol is cholesterol.

14. A process of any one of the preceding claims further comprising loading the liposomes with a therapeutic or diagnostic agent.

15. A process of Claim 14, wherein the therapeutic agent is an antineoplastic agent.

16. A process of Claim 14, wherein the antineoplastic agent is selected from doxorubicin hydrochloride, daunorubicin hydrochloride, and epirubicin hydrochloride.

17. A process of Claim 16, wherein the antineoplastic agent is doxorubicin hydrochloride.

18. A process of Claim 17, wherein phospholipid is distearoylphosphatidyl choline and the sterol is cholesterol.

19. A process of Claim 18, wherein the doxorubicin concentration encapsulated in the liposomes is from 1mM to 10mM expressed as doxorubicin hydrochloride.

20. A process of Claim 19, wherein said doxorubicin concentration is 3mM - 7mM.

21. A process of Claim 20, wherein said doxorubicin concentration is about 3.45mM.

22. A process of Claim 20, wherein said doxorubicin concentration is about 6.9mM.

23. A process of any one of Claims 18 to 22, wherein the molar ratio of distearoylphosphatidyl choline to cholesterol is from 1:0.6 to 1:0.8.

24. A process of any one of Claims 18 to 23, wherein the molar ratio of encapsulated doxorubicin (as hydrochloride) to distearoylphosphatidyl choline is from 1:2 to 1:15.

25. A process of Claim 24, wherein the molar ratio of encapsulated doxorubicin (as hydrochloride) to distearoylphosphatidyl choline is about 1:3.5.

26. A process of any one of Claims 18 to 25, wherein

the liposomes in the hydrated liposomal composition are sized;
extraliposomal ammonium sulphate is removed from the sized liposomal composition using a sucrose-histidine buffer solution comprising histidine hydrochloride and sucrose;
the liposomal composition freed of extraliposomal ammonium sulphate is admixed with a solution of at least 25mM doxorubicin hydrochloride dissolved in a sucrose-histidine buffer solution comprising histidine hydrochloride and sucrose to provide a doxorubicin loaded liposomal composition; and extraliposomal doxorubicin hydrochloride is removed from the liposomal composition.

27. A process of Claim 26, wherein, the molar ratio of sucrose to histidine hydrochloride in the isotonic buffer used to remove extraliposomal ammonium sulphate is 29:0.1 to 29:10.

28. A process of Claim 24 or Claim 25, wherein the sucrose concentration is from 0.1M to 0.5M.

29. A process of any one of Claims 26 to 28, wherein the concentration of histidine hydrochloride is from 8 to 12 mM.

30. A liposome obtainable by a process of Claim 2.

31. A liposome of Claim 30, wherein the process is as defined in any one of claims 4 to 30 when dependent directly or indirectly on Claim 3.

32. A long circulating non-pegylated liposomal doxorubicin composition for parenteral administration comprising, non pegylated doxorubicin liposomes of Claim 30 or Claim 31, histidine hydrochloride, and sucrose.

33. A long circulating non-pegylated liposomal doxorubicin composition for parenteral administration comprising doxorubicin non-pegylated liposomes of Claim 30 or Claim 31, histidine hydrochloride, and sucrose, wherein the doxorubicin non-pegylated liposomes comprise a phospholipid, cholesterol and sucrose.

34. A composition of Claim 33, wherein the liposome size and/or components are as defined in any one of Claims 5, 9, 10, 11, 12, 13, 18 to 25, 28 and 29.

35. A composition of any one of Claims 31 to 34, wherein the doxorubicin (as hydrochloride) is present at about 2mg/ml; the molar ratio of doxorubicin to phospholipid is about 1:3.5; and the ratio of phospholipid to cholesterol is about 1:0.7.

36. A composition of any one of Claims 31 to 34, wherein the doxorubicin (as hydrochloride) is present at about 4mg/ml, and the molar ratio of doxorubicin to phospholipid is about 1:3.5, and the ratio of phospholipid to cholesterol is about 1:0.7.

**Patentansprüche**

1. Verfahren zur Herstellung von lange zirkulierenden, nicht pegylierten Liposomen, bei dem:

   - ein oder mehrere Phospholipide und ein oder mehrere Sterine in einem Lösungsmittel oder einer Mischung von Lösungsmitteln aufgelöst werden,
   - die resultierenden Lipide hydratisiert werden und
   - das Lösungsmittel (die Lösungsmittel) vor oder nach der Hydratation entfernt werden,

   wobei die Hydratation mit einem wässrigen Hydratationsmedium in einer Menge im Bereich von 10 bis 35 ml je mmol in der Lipidlösung vorhandenem Phospholipid durchgeführt wird, um nicht pegylierte Liposome zu bilden, **dadurch gekennzeichnet, dass** das wässrige Hydratationsmedium Sucrose und nicht weniger als 125 mmol/l Ammoniumsulfat umfasst.

2. Verfahren nach Anspruch 1, bei dem die Sucrosekonzentration in dem wässrigen Hydratationsmedium 0,1 M bis 0,5 M beträgt.

3. Verfahren nach Anspruch 2, bei dem die Sucrosekonzentration 0,25 M bis 0,3 M beträgt.

4. Verfahren nach Anspruch 3, bei dem die Sucrosekonzentration etwa 0,27 M beträgt.

5. Verfahren nach einem der vorhergehenden Ansprüche, bei dem die Liposomen in der Liposomenzusammensetzung auf 0,06 $\mu$m bis 0,16 $\mu$m größenbemessen sind.

6. Verfahren nach einem der vorhergehenden Ansprüche, bei dem extraliposomales Hydratationssalz unter Verwendung einer Dialysepufferlösung aus der Liposomenzusammensetzung entfernt wird.

7. Verfahren nach einem der vorhergehenden Ansprüche, bei dem die Menge des verwendeten wässrigen Hydratationsmediums 20 bis 35 ml je mmol Phospholipid in der Lipidlösung beträgt.

8. Verfahren nach Anspruch 7, bei dem die Menge von verwendetem wässrigen Hydratationsmedium etwa 30 ml je mmol Phospholipid in der Lipidlösung beträgt.

9. Verfahren nach einem der vorhergehenden Ansprüche, bei dem molare Verhältnis von Phospholipid zu Sterin 1:0,1 bis 1:2 beträgt.

10. Verfahren nach Anspruch 9, bei dem das molare Verhältnis von Phospholipid zu Sterin etwa 1:0,7 beträgt.

11. Verfahren nach einem der vorhergehenden Ansprüche, bei dem das Phospholipid ausgewählt ist aus Distearoylphosphatidylcholin (DSPC), Dipalmitoylphosphatidylcholin (DPPC), hydriertem Soja-Phosphatidylcholin (HSPC) und Derivaten dieser Phospholipide.

12. Verfahren nach einem der vorhergehenden Ansprüche, bei dem das Phospholipiddistearoyl Phosphatidylcholin (DSPC) ist.

13. Verfahren nach einem der vorhergehenden Ansprüche, bei dem Sterin Cholesterin ist.

14. Verfahren nach einem der vorhergehenden Ansprüche, bei dem die Liposomen ferner mit einem therapeutischen oder diagnostischen Mittel beladen werden.

15. Verfahren nach Anspruch 14, bei dem das therapeutische Mittel ein antineoplastisches Mittel ist.

16. Verfahren nach Anspruch 14, bei dem das antineoplastische Mittel ausgewählt ist aus Doxorubicindihydrochlorid, Daunorubicinhydrochlorid und Epirubicinhydrochlorid.

17. Verfahren nach Anspruch 16, bei dem das antineoplastische Mittel Doxorubicinhydrochlorid ist.

18. Verfahren nach Anspruch 17, bei dem das Phospholipid Distearoylphosphatidylcholin ist und das Sterin Cholesterin ist.

19. Verfahren nach Anspruch 18, bei dem die Konzentration von in den Liposomen verkapseltem Doxorubicin 1 mM bis 10 mM beträgt, angegeben als Doxorubicinhydrochlorid.

20. Verfahren nach Anspruch 19, bei dem die Doxorubicinkonzentration 3 mM-7 mM beträgt.

21. Verfahren nach Anspruch 20, bei dem die Doxorubicinkonzentration etwa 3,45 mM beträgt.

22. Verfahren nach Anspruch 20, bei dem die Doxorubicinkonzentration etwa 6,9 mM beträgt.

23. Verfahren nach einem der Ansprüche 18 bis 22, bei dem das molare Verhältnis von Distearoylphosphatidylcholin zu Cholesterin 1:0,6 bis 1:0,8 beträgt.

24. Verfahren nach einem der Ansprüche 18 bis 23, bei dem das molare Verhältnis von verkapseltem Doxorubicin (als Hydrochlorid) zu Distearoylphosphatidylcholin 1:2 bis 1:15 beträgt.

25. Verfahren nach Anspruch 14, bei dem das molare Verhältnis von verkapseltem Doxorubicin (als Hydrochlorid) zu Distearoylphosphatidylcholin etwa 1:3,5 beträgt.

26. Verfahren nach einem der Ansprüche 18 bis 25, bei dem

- die Liposomen in der hydratisierten Liposomenzusammensetzung größenbemessen sind,
- extraliposomales Ammoniumsulfat unter Verwendung einer Sucrose-Histidin-Pufferlösung, die Histidinhydrochlorid und Sucrose umfasst, aus der Zusammensetzung von größenbemessenen Liposomen entfernt wird,
- die von extraliposomalem Ammoniumsulfat befreite Liposomenzusammensetzung mit einer Lösung von mindestens 25 mM Doxorubicinhydrochlorid, gelöst in einer Sucrose-Histidine-Pufferlösung, die Histidinhydrochlorid und Sucrose umfasst, vermischt wird, um eine mit Doxorubicin beladene Liposomenzusammensetzung bereitzustellen, und extraliposomales Doxorubicinhydrochlorid aus der Liposomenzusammensetzung entfernt wird.

27. Verfahren nach Anspruch 26, bei dem das molare Verhältnis von Sucrose zu Histidinhydrochlorid in dem zur Entfernung vom extraliposomalem Ammoniumsulfat verwendeten isotonischen Puffer 29:0,1 bis 29:10 beträgt.

28. Verfahren nach Anspruch 24 oder Anspruch 25, bei dem die Sucrosekonzentration 0,1 M bis 0,5 M beträgt.

29. Verfahren nach einem der Ansprüche 26 bis 28, bei dem die Konzentration von Histidinhydrochlorid 8 bis 12 mM beträgt.

30. Liposomen, erhältlich durch ein Verfahren gemäß Anspruch 2.

31. Liposom nach Anspruch 30, wobei das Verfahren wie in einem der Ansprüche 4 bis 30 definiert ist, wenn sie direkt oder indirekt auf Anspruch 3 bezogen sind.

32. Zusammensetzung von lange zirkulierendem nicht pegyliertem liposomalen Doxorubicin zur parenteralen Verabreichung, die nicht pegylierte Doxorubicinliposomen gemäß Anspruch 30 oder Anspruch 31, Histidinhydrochlorid und Sucrose enthält.

33. Zusammensetzung von lange zirkulierendem nicht pegyliertem liposomalen Doxorubicin zur parenteralen Verabreichung, die nicht pegylierte Doxorubicinliposomen gemäß Anspruch 30 oder Anspruch 31, Histidinhydrochlorid und Sucrose umfasst, wobei die Doxorubicin-nicht-pegylierten Liposomen Phospholipid, Cholesterin und Sucrose umfassen.

34. Zusammensetzung nach Anspruch 33, wobei die Liposomengröße und/oder -komponenten wie in einem der Ansprüche 5, 9, 10, 11, 12, 13, 18 bis 25, 28 und 29 definiert sind.

35. Zusammensetzung nach einem der Ansprüche 31 bis 34, wobei das Doxorubicin (als Hydrochlorid) mit etwa 2 mg/ml vorhanden ist, das molare Verhältnis von Doxorubicin zu Phospholipid etwa 1:3,5 beträgt und das Verhältnis von Phospholipid zu Cholesterin etwa 1:0,7 beträgt.

36. Zusammensetzung nach einem der Ansprüche 31 bis 34, wobei das Doxorubicin (als Hydrochlorid) mit etwa 4 mg/ml vorhanden ist, und das molare Verhältnis von Doxorubicin zu Phospholipid etwa 1:3,5 beträgt, und das Verhältnis von Phospholipid zu Cholesterin etwa 1:0,7 beträgt.

**Revendications**

1. Procédé pour la préparation de liposomes non pégylés à longue durée de circulation, comprenant :

   la dissolution d'un ou plusieurs phospholipides et d'un ou plusieurs stérols dans un solvant ou mélange de solvants,
   l'hydratation des lipides résultants et
   l'élimination dudit/desdits solvant(s) avant ou après ladite hydratation,
   ladite hydratation étant avec un milieu aqueux d'hydratation en une quantité dans la plage de 10 à 35 ml par mmole de phospholipide présent dans la solution lipidique, pour la formation de liposomes non pégylés, **caractérisé en ce que** le milieu aqueux d'hydratation comprend du saccharose et au moins 125 mmoles/litre de sulfate d'ammonium.

2. Procédé selon la revendication 1, dans lequel la concentration de saccharose dans le milieu aqueux d'hydratation vaut de 0,1 M à 0,5 M.

3. Procédé selon la revendication 2, dans lequel la concentration de saccharose vaut de 0,25 M à 0,3 M.

4. Procédé selon la revendication 3, dans lequel la concentration de saccharose est de 0,27 M.

5. Procédé selon l'une quelconque des revendications précédentes, dans lequel les liposomes dans la composition de liposomes sont calibrés à une taille de 0,06 $\mu$m à 0,16 $\mu$m.

6. Procédé selon l'une quelconque des revendications précédentes, dans lequel on élimine de la composition de liposomes le sel d'hydratation extraliposomique, à l'aide d'une solution tampon pour dialyse.

7. Procédé selon l'une quelconque des revendications précédentes, dans lequel la quantité de milieu aqueux d'hydratation utilisé vaut de 20 à 35 ml par mmole de phospholipide dans la solution de lipide.

8. Procédé selon la revendication 7, dans lequel la quantité de milieu aqueux d'hydratation utilisé est d'environ 30 ml par mmole de phospholipide dans la solution lipidique.

9. Procédé selon l'une quelconque des revendications précédentes, dans lequel le rapport molaire du phospholipide au stérol vaut de 1:0,1 à 1:2.

10. Procédé selon la revendication 9, dans lequel le rapport molaire du phospholipide au stérol est d'environ 1:0,7.

11. Procédé selon l'une quelconque des revendications précédentes, dans lequel le phospholipide est choisi parmi la distéaroylphosphatidylcholine (DSPC), la dipalmitoylphosphatidylcholine (DPPC), la phosphatidylcholine de soja hydrogénée (HSPC) et des dérivés de ces phospholipides.

12. Procédé selon l'une quelconque des revendications précédentes, dans lequel le phospholipide est la distéaroylphosphatidylcholine (DSPC).

13. Procédé selon l'une quelconque des revendications précédentes, dans lequel le stérol est le cholestérol.

14. Procédé selon l'une quelconque des revendications précédentes, comprenant en outre la charge des liposomes avec un agent thérapeutique ou un agent de diagnostic.

15. Procédé selon la revendication 14, dans lequel l'agent thérapeutique est un agent antinéoplasique.

16. Procédé selon la revendication 14, dans lequel l'agent antinéoplasique est choisi parmi le chlorhydrate de doxorubicine, le chlorhydrate de daunorubicine et le chlorhydrate d'épirubicine.

17. Procédé selon la revendication 16, dans lequel l'agent antinéoplasique est le chlorhydrate de doxorubicine.

18. Procédé selon la revendication 17, dans lequel le phospholipide est la distéaroylphosphatidylcholine et le stérol est le cholestérol.

19. Procédé selon la revendication 18, dans lequel la concentration de la doxorubicine encapsulée dans les liposomes vaut de 1 mM à 10 mM, exprimée en tant que chlorhydrate de doxorubicine.

20. Procédé selon la revendication 19, dans lequel ladite concentration de doxorubicine vaut de 3 mM à 7 mM.

21. Procédé selon la revendication 20, dans lequel ladite concentration de doxorubicine est d'environ 3,45 mM.

22. Procédé selon la revendication 20, dans lequel ladite concentration de doxorubicine est d'environ 6,9 mM.

23. Procédé selon l'une quelconque des revendications 18 à 22, dans lequel le rapport molaire de la distéaroylphosphatidylcholine au cholestérol vaut de 1:0,6 à 1:0,8.

24. Procédé selon l'une quelconque des revendications 18 à 23, dans lequel le rapport molaire de la doxorubicine encapsulée (sous forme de chlorhydrate) à la distéaroylphosphatidylcholine vaut de 1:2 à 1:15.

25. Procédé selon la revendication 24, dans lequel le rapport molaire de la doxorubicine encapsulée (sous forme de chlorhydrate) à la distéaroylphosphatidylcholine est d'environ 1:3,5.

26. Procédé selon l'une quelconque des revendications 18 à 25, dans lequel

les liposomes dans la composition hydratée de liposomes sont calibrés ;
on élimine le sulfate d'ammonium extraliposomique de la composition de liposomes calibrés, en utilisant une solution tampon saccharose-histidine comprenant du chlorhydrate d'histidine et du saccharose ;
on mélange la composition de liposomes, libérée du sulfate d'ammonium extraliposomique, avec une solution de chlorhydrate de doxorubicine au moins 25 mM dissous dans une solution tampon saccharose-histidine comprenant du chlorhydrate d'histidine et du saccharose, pour produire une composition de liposomes chargés de doxorubicine ; et on élimine de la composition de liposomes le chlorhydrate de doxorubicine extraliposomique.

27. Procédé selon la revendication 26, dans lequel le rapport molaire du saccharose au chlorhydrate d'histidine dans le tampon isotonique utilisé pour l'élimination du sulfate d'ammonium extraliposomique vaut de 29:0,1 à 29:10.

28. Procédé selon la revendication 24 ou la revendication 25, dans lequel la concentration de saccharose vaut de 0,1 M à 0,5 M.

29. Procédé selon l'une quelconque des revendications 26 à 28, dans lequel la concentration du chlorhydrate d'histidine vaut de 8 à 12 mM.

**30.** Liposome pouvant être obtenu par un procédé selon la revendication 2.

**31.** Liposome selon la revendication 30, dans lequel le procédé est tel que défini dans l'une quelconque des revendications 4 à 30, dépendant directement ou indirectement de la revendication 3.

**32.** Composition de liposomes non pégylés à longue durée de circulation, pour administration parentérale, comprenant des liposomes non pégylés à doxorubicine selon la revendication 30 ou la revendication 31, du chlorhydrate d'histidine et du saccharose.

**33.** Composition de liposomes non pégylés à longue durée de circulation, pour administration parentérale, comprenant des liposomes non pégylés à doxorubicine selon la revendication 30 ou la revendication 31, du chlorhydrate d'histidine et du saccharose, dans laquelle les liposomes non pégylés à doxorubicine comprennent un phospholipide, du cholestérol et du saccharose.

**34.** Composition selon la revendication 33, dans laquelle la taille et/ou les composants des liposomes sont tels que définis dans l'une quelconque des revendications 5, 9, 10, 11, 12, 13, 18 à 25, 28 et 29.

**35.** Composition selon l'une quelconque des revendications 31 à 34, dans laquelle la doxorubicine (sous forme de chlorhydrate) est présente à raison d'environ 2 mg/ml, le rapport molaire de la doxorubicine au phospholipide est d'environ 1:3,5 et le rapport du phospholipide au cholestérol est d'environ 1:0,7.

**36.** Composition selon l'une quelconque des revendications 31 à 34, dans laquelle la doxorubicine (sous forme de chlorhydrate) est présente à raison d'environ 4 mg/ml, et le rapport molaire de la doxorubicine au phospholipide est d'environ 1:3,5 et le rapport du phospholipide au cholestérol est d'environ 1:0,7.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6132763 A
- US 4769250 A
- US 4501728 A
- US 4920016 A
- US 6083530 A
- US 5013556 A
- US 5676971 A
- WO 8806442 A
- WO 8500968 A
- US 5316771 A
- US 4235871 A
- EP 0561424 A
- US 4880635 A

**Non-patent literature cited in the description**

- *Cancer Investigation,* 2001, vol. 19 (4), 424-436
- **Alberto A. Gabizon.** *Cancer Investigation,* 2001, vol. 19 (4), 424-436
- **Kenneth B. Gordon.** *Cancer,* 1995, vol. 75 (8), 2169-2173
- *Clinical Cancer Research,* 1999, 3645-3652
- **Zhigaltsev et al.** *J. Liposome Res.,* vol. 11 (1), 55-71
- Guide for Care and Use of Laboratory Animals. National Academy Press, 1996